(19) Europäisches Patentamt
European Patent Office
Office européen des brevets

(11) Veröffentlichungsnummer: **0 067 792**
**B1**

(12) # EUROPÄISCHE PATENTSCHRIFT

(45) Veröffentlichungstag der Patentschrift: **26.02.86**

(21) Anmeldenummer: **82810243.4**

(22) Anmeldetag: **07.06.82**

(51) Int. Cl.⁴: **C 07 D 263/56**, C 07 D 498/04, C 07 C 131/00, C 07 D 249/24, C 07 D 249/20, C 07 D 271/10, C 07 D 261/08, C 07 D 249/06, C 07 D 471/04, C 07 D 237/14, D 06 L 3/12

(54) **Stilbenverbindungen.**

(30) Priorität: **12.06.81 CH 3880/81**

(43) Veröffentlichungstag der Anmeldung
**22.12.82 Patentblatt 82/51**

(45) Bekanntmachung des Hinweises auf die Patenterteilung:
**26.02.86 Patentblatt 86/9**

(84) Benannte Vertragsstaaten:
**CH DE FR GB IT LI**

(56) Entgegenhaltungen:
**EP - A - 0 002 042**
**EP - A - 0 031 796**
**EP - A - 0 033 459**
**DE - A - 2 709 924**

(73) Patentinhaber: **CIBA-GEIGY AG, Klybeckstrasse 141, CH-4002 Basel (CH)**

(72) Erfinder: **Weber, Kurt, Dr., Rennweg 98, CH-4052 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER. STOCKHOLM 1986

EP 0 067 792 B1

**Beschreibung**

Die vorliegende Erfindung betrifft neue Stilbenverbindungen, Verfahren zu deren Herstellung sowie deren Verwendung zum optischen Aufhellen von natürlichen und synthetischen organischen Materialien.

Stilbenverbindungen sind z.B. aus der US-Patentschrift 3,984,399 sowie auch aus den DE-A-2 709 924, EP-A-0-002 042, EP-A-0031 796 und EP-A-0033 459 bekannt. Aufgabe der vorliegenden Erfindung war, neue optische Aufheller mit besseren Aufzieheigenschaften zu beschaffen.

Es wurde nun gefunden, dass bestimmte Stilbenverbindungen mit einem p-(Oximäther-rest) ausgiebiger sind als bis jetzt bekannte Stilbenverbindungen.

Die neuen Stilbenverbindungen entsprechen der Formel

(1) 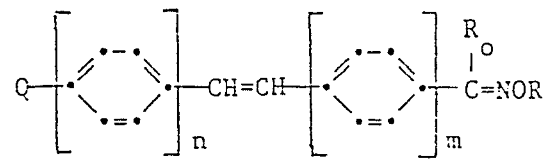 ,

worin Q Wasserstoff; einen monocyclischen 5- oder 6-gliedrigen unsubstituierten oder nicht-chromophor substituierten 0,1 oder 2 ankondensierte Benzolringe aufweisenden aromatischen heterocyclischen Ring; einen bicyclischen 9-gliedrigen aromatischen heterocyclischen Ring oder den Rest $-CH=C(R_1)(R_2)$, worin $R_1$ für Wasserstoff, unsubstituiertes oder nicht-chromophor substituiertes $C_{1-6}$ -Alkyl und $R_2$ für Alkoxycarbonyl mit insgesamt 2 bis 7 C-Atomen, Cyano, Carboxy Carbamoyl, phosphonsäuredialkylester, $C_{1-6}$-Alkyl- oder Arylsulfonyl stehen oder Q auch

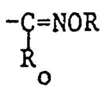

bedeuten kann, $R_0$ Wasserstoff oder $C_{1-6}$ -Alkyl, R unsubstituiertes oder nicht-chromophor substituiertes $C_{1-6}$ -Alkyl, $C_{2-5}$ -Alkenyl, $C_{2-5}$ -Alkinyl, Di-$C_{1-4}$ -alkylamino-$C_{1-4}$ -alkyl, Hydroxy-$C_{1-4}$-alkyl, phenyl-$C_{1-4}$ -alkyl oder Cycloalkyl mit 5 bis 12 Ringgliedern und m und n unabhängig voneinander die Zahl 1 oder 2 bedeuten.

Nicht-chromophore Substituenten sind z.B. Halogen, unsubstituiertes oder substituiertes Alkyl, unsubstituiertes oder substituiertes Alkenyl, Aryl, Aralkyl, unsubstituiertes oder substituiertes Alkoxy, Alkoxycarbonyl, unsubstituiertes oder substituiertes Aminocarbonyl, Cyano, Alkylsulfonyl, Alkoxysulfonyl, unsubstituiertes oder substituiertes Aminosulfonyl, Acyl, Acylamino, Aryloxy, Aralkoxy, Alkenyloxy, Aryloxycarbonyl, Aralkoxycarbonyl, Carboxy, Acyloxy oder Trifluormethyl.

Alkyl ist besonders $C_{1-4}$ -Alkyl, das durch Hydroxy, $C_{1-4}$ -Alkoxy, Cyano, Carboxy, $C_{2-5}$ -Alkoxycarbonyl, Aminocarbonyl oder Chlor monosubstituiert sein kann.

Alkenyl ist insbesondere $C_{2-5}$ -Alkenyl, das durch Hydroxy, $C_{1-4}$-Alkoxy, Cyano, Carboxy, $C_{1-4}$ -Alkoxycarbonyl oder Chlor monosubstituiert sein kann.

Halogen ist insbesondere Fluor, Chlor oder Brom, vorzugsweise Chlor.

Aryl ist besonders Phenyl oder durch $C_{1-4}$ -Alkyl, Chlor, Brom oder $C_{1-4}$ -Alkoxy substituiertes Phenyl.

Aralkyl ist insbesondere Phenyl-$C_{1-4}$ -alkyl, das im Phenylkern noch durch Chlor, Methyl oder Methoxy substituiert sein kann.

Alkoxy ist insbesondere $C_{1-4}$ -Alkoxy oder einen Rest $-(OCH_2-CH_2)_p-OY$ wobei Y Wasserstoff oder $C_{1-4}$ -Alkyl und ganze Zahl von 1 bis 20 bedeutet.

Acyl ist insbesondere $C_{2-5}$ -Alkanoyl, $C_{1-4}$ -Alkylsulfonyl, Benzoyl, durch Chlor, Methyl oder Methoxy substituiertes Benzoyl, Phenylsulfonyl oder durch Chlor, Methyl oder Methoxy substituiertes Phenylsulfonyl.

Als Substituenten der Aminocarbonyl- und Aminosulfonylreste kommen besonders $C_{1-4}$ -Alkyl, Phenyl, Phenyl-$C_{1-4}$ -alkyl oder durch Chlor, Methyl oder Methoxy substituiertes Phenyl in Betracht.

Als aromatische heterocyclische Ringe kommen die folgenden in Betracht:

Benzoxazol, Benzimidazol, v-Triazol, 1,3,4-Oxdiazol, Pyrimidin, Pyridazinon, Pyrazin, s-Triazin, 1,3,4-Oxdiazolon, 1,2,4-Oxdiazol, Pyrazol, Isoxazol, Benzisoxazol, Oxazolo[5.4-b]pyridin, Oxazolo-[5,4-e]pyridin und 1,2,4-Tri-azolo[1,5-a]pyridin.

Als Substituenten von Benzoxazol- und Benzimidazolringen kommen Chlor, $C_1$ -$C_4$ -Alkyl, Phenyl-$C_1$ -$C_3$ -Alkyl, Cyclohexyl, phenyl, $C_1$ -$C_4$-Alkoxy, $C_1$ -$C_4$ -Alkylsulfonyl, $C_1$ -$C_4$ -Alkoxycarbonyl, Cyano oder Carboxy oder zusammen mit einem zweiten Substituent einen gegebenenfalls durch 1 bis 4 Methylgruppen substituierten ankondensierten Cylopentan-, Cyclohexan- oder Benzolring, in Betracht. Benzimidazolreste können ausserdem in 1-Stellung noch durch $C_{1-4}$ -Alkyl oder Phenyl substituiert sein.

v-Triazolylreste können durch 1 oder 2 $C_{1-4}$ -Alkylreste, einen Chlor $C_{1-4}$ -Alkylrest einen $C_{1-4}$ -Alkoxy, einen Cyanorest, einen $COOC_{1-4}$-Alkylrest, 1 oder 2 Phenylreste oder 1 Styrylrest substituiert sein. Der v-Triazolylrest

kann auch einen unsubstituierten oder durch $C_{1-4}$ -Alkoxy substituierten ankondensierten Benzolring oder einen ankondensierten Naphthalinring aufweisen.

1,2,4- und 1,3,4-Oxdiazolreste können durch unsubstituiertes $C_{1-4}$-Alkyl, nicht-chromophor substituiertes $C_{1-4}$ -Alkyl, unsubstituiertes Phenyl, Styryl, Biphenylyl oder Naphthyl oder durch $C_{1-4}$ -Alkyl, $C_{1-4}$ -Alkoxy, $C_{1-4}$ -Alkoxycarbonyl, Cyano oder Chlor substituiertes Phenyl, Styryl, Biphenylyl oder Naphthyl, substituiert sein.

Pyrimidinreste können unsubstituiert oder substituiert sein. Als Substituenten sind z.B. zu erwähnen: $C_{1-4}$ -Alkyl, unsubstituiertes oder durch Chlor oder Methyl substituiertes Phenyl, $C_{1-4}$ -Alkoxy, $C_{3-8}$ -Alkoxyalkoxy, unsubstituiertes oder durch Chlor oder Methyl substituiertes Phenoxy, Chlor, $C_{1-4}$ -Alkylthio, Phenylthio, $C_{1-4}$-Alkylamino, Di-$C_{1-4}$ -alkylamino, Morpholino, Piperidino, Piperazino, Pyrrolidino oder Anilino.

Ein Pyridazinonrest kann in 3-Stellung durch $C_{1-4}$ -alkyl oder unsubstituiertes oder durch Chlor oder Methyl substituiertes Phenyl substituiert sein.

Pyrazinreste können unsubstituiert oder in 5-Stellung substituiert sein durch z.B. $C_{1-4}$ -Alkoxy, $C_{3-8}$ -Alkoxyalkoxy, $C_{1-4}$ -Alkylthio,unsubstituiertes oder durch Chlor oder Methyl substituiertes Phenoxy oder Phenylthio; $C_{1-4}$ -Alkylamino, Di-$C_{1-4}$ -Alkylamino, Morpholino, Piperidino, Piperazino, Pyrrolidino oder Anilino.

Als Substituenten von s-Triazinresten kommen in Betracht z.B. Halogen, $C_{1-4}$ -Alkyl, $C_{-4}$ -Alkoxy, Phenyl, Aralkoxy, Cycloalkoxy, Aryloxy, besonders Phenoxy, $C_{1-4}$ -Alkylmercapto, $C_{1-4}$ -Alkylamino, Di-$C_{1-4}$-alkylamino, Morpholino, Piperidino, Piperazino, Pyrrolidino oder Arylamino besonders Phenylamino. .

Ein 1,2,4-Oxdiazolonrest kann durch $C_{1-4}$ -Alkyl oder unsubstituiertes oder durch $C_{1-4}$ -Alkyl, Halogen oder Cyano substituiertes Phenyl, substituiert sein.

Pyrazolreste weisen ein unsubstituiertes oder durch Chlor oder $C_{1-4}$ -Alkyl substituiertes Phenyl auf und können zusätzlich noch durch einen $C_{1-4}$ -Alkylrest substituiert sein.

Isoxazolreste sind durch ein unsubstituiertes oder durch $C_{1-4}$ -Alkoxy substituiertes Phenyl substituiert.

Als Cycloalkylreste R kommen besonders Cyclopentyl-, Cyclohexyl und Cycloheptylreste in Betracht.

Im Rahmen der Verbindungen der Formel (1) sind von Interesse solche der Formel

(2) 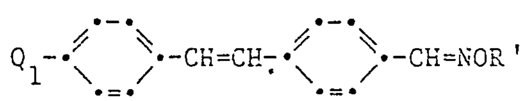 ,

worin R' $C_{1-4}$ -Alkyl Cyano-$C_{1-4}$ -Alkyl, $C_{1-4}$ -Alkoxy- $C_{2-4}$ -Alkyl, $C_{3-4}$ -Alkenyl, $C_{3-4}$ -Alkinyl oder $R_x$ OOC-$C_{1-4}$ -Alkyl, worin $R_x$ für Wasserstoff oder $_{1-4}$-Alkyl steht und $Q_1$ Phenyl oder den Rest -CH = C(R'$_1$)(R'$_2$),

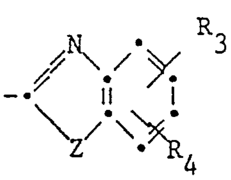 , 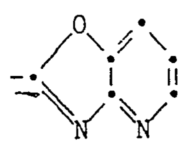 , 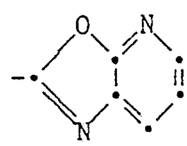 ,

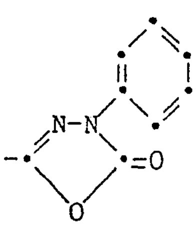 , 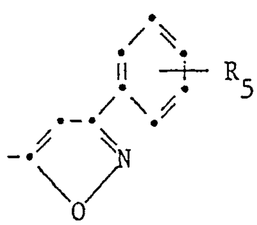 , 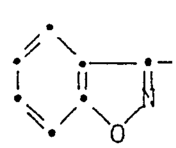 , 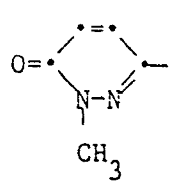 ,

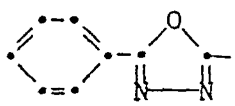 , 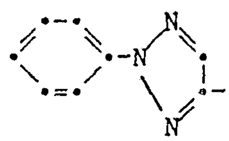 , 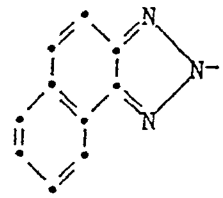

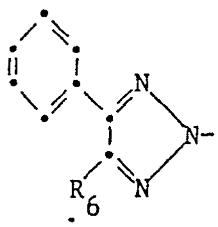 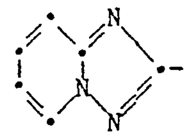 oder 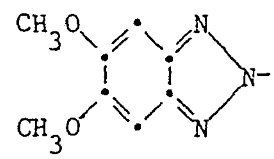

bedeuten, worin $R'_1$, Wasserstoff oder $C_{1-4}$-Alkyl, $R'_2$ Alkoxycarbonyl mit insg 2 bis 5 C-Atomen, Cyano, Carboxy, Carbamoyl, $C_{1-4}$-Alkylsulfonyl oder phenylsulfonyl, $R_3$ Wasserstoff, Chlor, $C_{1-4}$-Alkyl, Phenyl-$C_{1-3}$-alkyl Cyclohexyl, Phenyl, $C_{1-4}$-Alkoxy, $C_{1-4}$-Alkylsulfonyl, $C_{2-5}$-Alkoxycarbonyl, Cyano oder Carboxy oder zusammen mit $R_4$ einen unsubstituierten oder durch 1 bis 4 Methylgruppen substituierten ankondensierten Cyclopentan-, Cyclohexan- oder Benzolring, $R_4$ Wasserstoff, Chlor, $C_{1-4}$-Alkyl oder $C_{1-4}$-Alkoxy oder zusammen mit $R_3$ einen unsubstituierten oder durch 1 bis 4 Methylgruppen substituierten ankondensierten 1-Cyclopenten-, 1-Cyclohexen- oder Benzolring $R_5$ Wasserstoff oder Methoxy, $R_6$ Wasserstoff, $C_{1-4}$-Alkyl oder Alkoxycarbonyl mit insgesamt 2 bis 5 C-Atomen und Z O, S oder NX bedeuten, worin X für Wasserstoff, $C_{1-4}$-Alkyl oder Phenyl steht.

Bevorzugte Stilbenverbindungen entsprechen der Formel

(3) 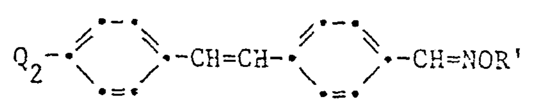 ,

worin R' die oben angegebene Bedeutung hat und $Q_2$ den Rest $-CH=C(R_1')(R_2'')$,

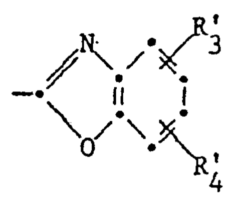 , 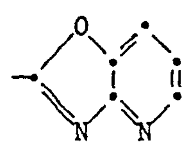 oder

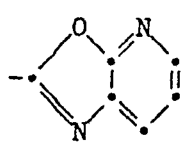

bedeutet, worin

$R_1'$ Wasserstoff oder $C_{1-4}$-Alkyl, $R_2''$-Alkoxycarbonyl mit insgesamt 2 bis 5 C-Atomen oder Cyano, $R_3'$ Wasserstoff, $C_{1-4}$-Alkyl, $C_{1-4}$-Alkoxy oder Alkoxycarbonyl mit insgesamt 2 bis 5 C-Atomen oder zusammen mit $R_4'$ einen ankondensierten Benzolring und $R_4'$ Wasserstoff oder $C_{1-4}$-Alkyl oder zusammen mit $R_3'$ einen ankondensierten Benzolring bedeuten.

Weitere bevorzugte Verbindungen der Formel (1) sind solche der Formel

(4) 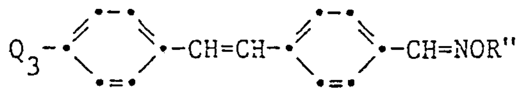 ,

worin R'' $C_{1-4}$-Alkyl, $C_{1-4}$-Alkoxy-$C_{2-4}$-Alkyl oder $C_{3-4}$-Alkenyl und $Q_3$ den Rest -CH=CH-CN, -CH=CH-COO$C_{1-4}$-Alkyl oder den Rest

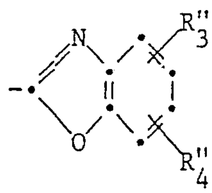

bedeuten, worin $R_3''$ für Wasserstoff, $C_{1-4}$-Alkyl, $C_{1-4}$-Alkoxy oder $C_{2-5}$-Alkoxycarbonyl und $R_4''$ für Wasserstoff oder $C_{1-4}$-Alkyl stehen.

Besonders bevorzugt sind die Verbindungen der Formel

(5) 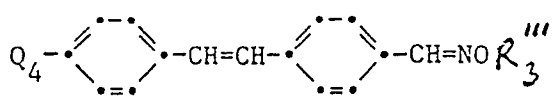 ,

worin $R_3'''$ $-CH_3$, $-C_2H_5$, $-CH_2CH_2OCH_3$ oder $-CH_2-CH=CH_2$ und $Q_4$ den Rest -CH=CH-CN, -CH=CH-COO$C_{1-2}$-Alkyl oder

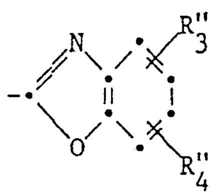

bedeutet, worin $R_3'''$ Wasserstoff oder $C_{1-4}$-Alkyl und $R_4'''$ Wasserstoff oder $C_{1-4}$-Alkyl bedeuten und besonders jene der Formeln

(6) 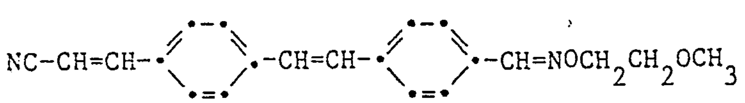 und

(7) 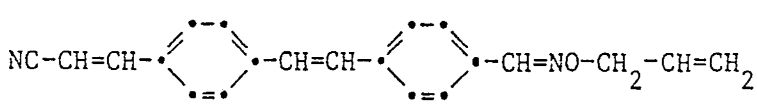 .

Die erfindungsgemässen Stilbenverbindungen der Formel (1) können dadurch hergestellt werden, dass man eine Carbonylverbindung der Formel

(8) 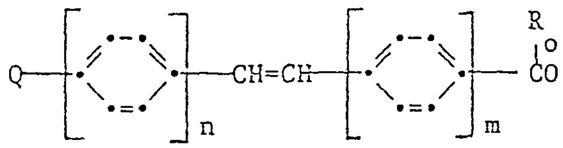 ,

worin Q, m und n die oben angegebene Bedeutung haben, mit einem O-substituierten Hydroxylamin der Formel

$$(9) \qquad H_2NOR \quad ,$$

worin R die oben angegebene Bedeutung hat, in einem gegenüber den Reaktionsteilnehmern inerten organischen Lösungsmittel umsetzt. Anstelle der freien Basen der Formel (9) kann man auch deren Salze mit vorzugsweise einer Halogenwasserstoffsäure, besonders HCl, in Anwesenheit einer die freie Base freisetzende Verbindung wie z.B. Natriumacetat, verwenden.

Als inerte organische Lösungsmittel verwendet man beispielsweise Alkohole wie Methanol, Aethanol, Isopropanol, Butanol, Glykol, Glykoläther wie 2-Methoxyäthanol, Aethylenglykolmonomethyläther, Aethylenglykolmonoäthyläther, Diäthylenglykolmonomethyl, -dimethyl-,-monoäthyl und -diäthyläther, Cyclohexanol, Cyclooctanol, ferner Aether wie Diisopropyläther, Dioxan, Tetrahydrofuran, weiterhin Formamide oder N-Methylpyrrolidon.

Die O-substituierten Hydroxylamine der Formel(9) sind bekannten Verbindungen oder können nach an sich bekannten Methoden hergestellt werden, wie z.B. in Houben-Weyl, Bd X/I, Seiten 1181 ff. (1971) angegeben.

Man arbeitet vorzugsweise im Temperaturbereich von 0 bis zum Siedepunkt der verwendeten Lösungsmittels.

An den Reaktionsprodukten des vorstehenden Verfahrens können selbstverständlich noch weitere an sich bekannte Umwandlungen vorgenommen werden wie Halogenierungen, funktionelle Abwandlungen von Carboxylgruppen, Einführung von Chlormethylgruppen oder Austausch von Halogenatomen gegen Cyanogruppen.

Die Stilbenverbindungen der Formel (1) können auch dadurch hergestellt werden, dass man ei-n Mol einer Verbindung der Formel

(10) 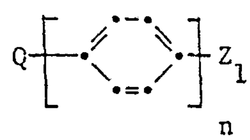

mit einem Mol einer Verbindung der Formel

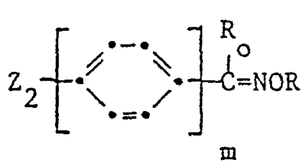

in Anwesenheit einer starken Base und eines polaren Lösungsmittels umsetzt, wobei Q, n, $R_o$, R und m die oben angegebene Bedeutung haben und eines der Symbole Z und Z eine HOC-Gruppe und das andere eine Gruppierung der Formel

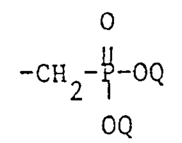 , 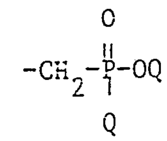 , 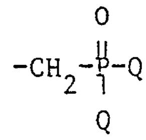 oder 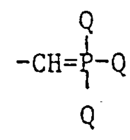

bedeuten, worin Q einen unsubstituierten oder substituierten Alkylrest, mit 1 bis 6 C-Atomen, einen Arylrest, vorzugswei-se Phenylrest, einen Cycloalkylrest, vorzugsweise einen Cyclohexylrest oder einen Aralkylrest, vorzugsweise Benzylrest, darstellt.

Als Lösungsmittel kommen z.B. Alkohole wie Methanol, Aethanol, Isopropanol, Butanol, Glykole, Glykoläther wie 2-Methoxyäthanol, Hexanole, Cyclohexanol und Cyclooctanol, ferner Aether wie Diisopropyläther, Tetrahydrofuran und Dioxan sowie Dimethylsulfoxyd, Formamid und N-Methylpyrrolidon in Betracht. Besonders geeignet sind polare organische Lösungsmittel wie Dimethylformamid und Dimethylsulfoxyd. Auch in wässriger Lösung lassen sich einige der Umsetzungen durchführen.

Die Temperatur, bei welcher die Umsetzung durchgeführt wird, kann in weiten Grenzen schwanken. Sie wird bestimmt:

α) durch die Beständigkeit des verwendeten Lösungsmittels gegenüber den Reaktionsteilnehmern, insbesondere gegenüber den stark basischen Alkaliverbindungen,

ß) durch die Reaktivität der Kondensationspartner und

γ) durch die Wirksamkeit der Kombination Lösungsmittel-Base als Kondensationsmittel.

In der praxis kommen hiernach im allgemeinen Temperaturen zwischen etwa 10° und 100°C in Betracht, insbesondere, wenn Dimethylformamid oder Dimethylsulfoxyd als Lösungsmittel verwendet werden. Der Temperatur-Vorzugsbereich liegt bei 20° bis 60°C.

Als starke Basen kommen vor allem die Hydroxyde, Amide und Alkoholate (vorzugsweise solche primärer, 1 bis 4 Kohlenstoffatome enthaltender Alkohole) der Alkalimetalle in Betracht, wobei aus ökonomischen Gründen solche des Lithiums, Natriums und Kaliums von vorwiegendem Intresse sind. Es können jedoch grundsätzlich und in besonderen Fällen auch Alkalisulfide und -carbonate, Arylalkaliverbindungen wie z.B. Phenyllithium oder stark basische Amine (einschliesslich Ammoniumbasen) z.B. Trialkylammoniumhydroxyde, mit Erfolg verwendet werden.

Die erfindungsgemässen Verbindungen zeigen in gelöstem oder feinverteiltem Zustande eine ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Hierfür seien beispielsweise, ohne dass durch die nachfolgende Uebersicht irgendeine Beschränkung hierauf ausgedrückt werden soll, die folgenden Gruppen von organischen Materialien,sofern eine optische Aufhellung derselben in Betracht kommt, genannt:

I. Synthetische organische hochmolokulare Materialien:

a) Polymerisationsprodukte auf Basis mindestens eine polymerisierbare Kohlenstoff-Kohlenstoff-Doppelbindung enthaltender organischer Verbindungen, d.h. deren Homo- oder Copolymerisate sowie deren Nachbehandlungsprodukte wie beispielsweise Vernetzungs-, Pfropfungsoder Abbauprodukte, Polymerisat-Verschnitte oder durch Modifizierung reaktionsfähiger Gruppen erhaltene Produkte, beispielsweise Polymerisate auf Basis von α,ß-ungesättigten Carbonsäuren oder Derivaten solcher Carbonsäuren, insbesondere von Acrylverbindungen (wie z.B. Acrylestern, Acrylsäure, Acrylnitril, Acrylamiden und deren Derivaten oder deren Methacryl-Analoga), von Olefin-Kohlenwasserstoffen (wie z.B. Aethylen, Propylen, Styrole oder Diene, ferner sogenannte ABS-Polymerisate), Polymerisate auf Basis von Vinyl- und Vinyliden-Verbindungen (wie z.B. Vinylchlorid, Vinylalkohol, Vinylidenchlorid),

b) Polymerisationsprodukte, die durch Ringöffnung erhältlich sind, z.B. Polyamide vom Polycaprolactam-Typ, ferner Polymere, die sowohl über Polyaddition als auch Polykondensation erhältlich sind, wie Polyäther oder Polyacetale,

c) Polykondensationsprodukte oder Vorkondensate auf Basis di oder polyfunktioneller Verbindungen mit kondensationsfähigen Gruppen, deren Homo- und Mischkondensationsprodukte sowie Produkte der Nachbehandlung, wie beispielsweise Polyester, insbesondere gesättigte (z.B. Aethylenglykolterephthalsäurepolyester) oder ungesättigte (z.B. Maleinsäure-Dialkohol-Polykondensate sowie deren Vernetzungsprodukte mit anpolymerisierbaren Vinylmonomeren), unverzweigte sowie verzweigte (auch auf Basis höherwertiger Alkohole, wie z.B. Alkydharze) Polyester, Polyamide (z.B. Hexamethylendiaminadipat), Maleinatharze, Melaminharze, deren Vorkondensate und Analoga, Polycarbonate, Silikone,

d) Polyadditionsprodukte wie Polyurethane (vernetzt und unvernetzt), Epoxidharze,

II. Halbsynthetische organische Materialien, z.B. Celluloseester verschiedener Veresterungsgrade (sogenanntes 2 1/2-Acetat, Triacetate) oder Celluloseäther, regenerierte Cellulose (Viskose, Kupferammoniak-Cellulose) oder deren Nachbehandlungsprodukte, Casein-Kunststoffe.

III. Natürliche organische Materialien animalischen oder vegetabilischen Ursprungs, beispielsweise auf Basis

von Cellulose oder Proteinen wie Baumwolle, Wolle, Leinen, Seide, natürliche Lackharze, Stärke, Casein.

Die optisch aufzuhellenden organischen Materialien können den verschiedenartigsten Verarbeitungszuständen (Rohstoffe, Halbfabrikate oder Fertigfabrikate) angehören. Sie können andererseits in Form der verschiedenartigsten geformten Gebilde vorliegen, d.h. beispielsweise als vorwiegend dreidimensional ausgedehnte Körper wie platten, Profile, Spritzgussformlinge, verschiedenartige Werkstücke, Schnitzel, Granulate oder Schaumstoffe, ferner als vorwiegend zweidimensional ausgebildete Körper wie Filme, Folien, Lacke, Ueberzüge, Imprägnierungen und Beschichtungen oder als vorwiegend eindimensional ausgebildete Körper wie Fäden, Fasern, Flocken, Drähte. Die besagten Materialien können andererseits auch in ungeformten Zuständen in den verschiedenartigsten homogenen oder inhomogenen Verteilungsformen, wie z.B. als pulver, Lösungen, Emulsionen, Dispersionen, Latices, Pasten oder Wachse vorliegen.

Fasermaterialien können beispielsweise als endlose Fäden (verstreckt oder unverstreckt), Stapelfasern, Flocken, Strangware, textile Fäden, Garne, Zwirne, Faservliese, Filze, Watten, Beflockungs-Gebilde oder als textile Gewebe oder textile Verbundsstoffe, Gewirke sowie als papiere, Pappen oder Papiermassen vorliegen.

Den erfindungsgemäss anzuwendenden Verbindungen kommt u.a. Bedeutung für die Behandlung von textilen organischen Materialien, insbesondere textilen Gewebe, zu. Sofern Fasern, welche als Stapelfasern oder Endlosfäden, in Form von Strangen, Geweben, Gewirken, Vliesen, beflockten Substraten oder Verbundstoffen vorliegen können, erfindungsgemäss optisch aufzuhellen sind, geschieht dies mit Vorteil in wassrigem Medium, worin die betreffenden Verbindungen in feinverteilter Form (Suspensionen, sogenannten Mikrodispersionen, gegebenenfalls Lösungen) vorliegen. Gegebenenfalls können bei der Behandlung Dispergier-, Stabilisier-, Netz- und weitere Hilfsmittel zugesetzt werden.

In Abhängigkeit vom verwendeten Aufheller-Verbindungstyp kann es sich als vorteilhaft erweisen, in neutralem oder alkalischem oder saurem Bade zu arbeiten. Die Behandlung wird üblicherweise bei Temperaturen von etwa 20 bis 140°C, beispielsweise bei Siedetemperatur des Bades oder in deren Nähe (etwa 90°C), durchgeführt. Für die erfindungsgemässe Veredlung textiler Substrate kommen auch Lösungen oder Emulsionen in organischen Lösungsmitteln in Betracht, wie die in der Färbereipraxis in der sogenannten Lösungsmittelfärberei (Foulard-Thermofixierapplikation, Ausziehfärbeverfahren in Färbemaschinen) praktiziert wird.

Die neuen optischen Aufhellmittel gemäss vorliegender Erfindung können ferner den Materialien vor oder während deren Verformung zugesetzt bzw. einverleibt werden. So kann man sie beispielsweise bei der Herstellung von Filmen, Folien (z.B. Einwalzen in Polyvinylchlorid in der Hitze) oder Formkörpern der pressmasse oder Spritzgussmasse beifügen.

Sofern die Formgebung voll- oder halbsynthetischer organischer Materialien durch Spinnverfahren bzw. über Spinnmassen erfolgt, können die optischen Aufheller nach folgenden Verfahren appliziert werden:

- Zugabe zu den Ausgangssubstanzen (z.B. Monomeren) oder Zwischenprodukten (z.B. Vorkondensaten, Praepolymeren), d.h. vor oder während der Polymerisation, Polykondensation oder Polyaddition,
- Aufpudern auf Polymerisatschnitzel oder Granulate für Spinnmassen,
- Badfärbung von polymerisatschnitzeln oder Granulaten für Spinnmassen,
- Dosierte Zugabe zu Spinnschmelzen oder Spinnlösungen,
- Applikation auf Spinnkabel vor dem Verstrecken.

Die neuen optischen Aufhellmittel gemäss vorliegender Erfindung können beispielsweise auch in folgenden Anwendungsformen eingesetzt werden:

a) Mischungen mit Farbstoffen (Nuancierung) oder Pigmenten (Farboder insbesondere z.B. Weisspigmente) oder als Zusatz zu Färbebädern, Druck-, Aetz- oder Reservepasten. Ferner auch zur Nachbehandlung von Färbungen, Drucken oder Aetzdrucken,

b) in Mischungen mit sogenannten "Carriern", Netzmitteln, Weichmachern, Quellmitteln, Antioxydantien, Lichtschutzmitteln, Hitzestabilisatoren, chemischen Bleichmitteln (Chlorit-Bleiche, Bleichbäder-Zusätze),

c) in Mischungen mit Vernetzern, Appreturmitteln (z.B. Stärke oder synthetischen Appreturen) sowie in Kombination mit den verschiedensten Textilveredlungsverfahren, insbesondere Kunstharzausrüstungen (z.B. Knitterfest-Ausrüstungen wie "wash-and-wear", "permanent-press", "no-iron"), ferner Flammfest-, Weichgriff-, Schmutzablöse ("anti-soiling") oder Antistatisch-Ausrüstungen oder antimikrobiellen Ausrüstungen,

d) Einarbeiten der optischen Aufhellmittel in polymere Trägermaterialien (Polymerisations-, Polykondensations- oder Polyadditionsprodukte) in gelöster oder dispergierter Form für Anwendung z.B. in Beschichtungs-, Imprägnier- oder Bindemitteln (Lösungen, Dispersionen, Emulsionen) für Textilien, Vliese, Papier, Leder,

e) als Zusätze zu sogenannten "master batches",

f) als Zusätze zu den verschiedensten industriellen produkten, um dieselben marktfähiger zu machen (z.B. Aspektverbesserung von Seifen, Waschmitteln, pigmenten),

g) in Spinnbadpräparationen, d.h. als Zusätze zu Spinnbädern wie sie zur Gleitfähigkeitsverbesserung für die Weiterverarbeitung von Synthese-Fasern verwendet werden, oder aus einem speziellen Bad vor der Verstreckung der Faser,

h) als Szintillatoren, für verschiedene Zwecke photographischer Art, wie z.B. für elektrophotographische Reproduktion oder Supersensibilisierung,

i) je nach Substitution als Laser-Farbstoffe.

Die erfindungsgemässen Verbindungen können auch in Kombination mit anderen optisch aufhellenden Verbindungen verwendet werden. Als zu mischende Verbindungen kommen z.B. die folgenden bekannten in Betracht: 1,4-Bis-(benzoxazol-2'-yl)-naphthalin, 4,4'-Bis-(äthoxy-carbonyl-vinyl)-stilben, 4,4'-Bis-(cyan-vinyl)-stilben, 1,4-Bis-(2'-cyano-styryl)-benzol, 1,5-Bis-(benzoxazol-2'-yl)-thiophen, 1-phenyl-4-(5',7'-dimethyl-benzoxazol-2'-yl)-stilben, 1,2-Bis-(5'-methyl-benzoxazol-2'-yl)-vinylen, 4-(benzoxazol-2'-yl)-4'-(3"-methyl-1",2",4"-oxadiazol-5"-yl)-stilbene und 2,4-Dimethoxytriazin-6-yl-pyren.

Die so erstellbaren Aufhellermischungen enthalten die erfindungsgemässe Verbindung und die bekannte im Verhältnis 1:9 bis 9:1, vorzugsweise 1:2 oder 2:1.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellende Verbindungen in solcher Konzentration enthalten, dass der gewünschte Aufhelleffekt erreicht wird.

In gewissen Fällen werden die Aufheller durch eine Nachbehandlung zur vollen Wirkung gebracht. Diese kann beispielsweise eine chemische (z.B. Säure-Behandlung), eine thermische (z.B. Hitze) oder eine kombinierte chemisch/thermische Behandlung darstellen. So verfährt man beispielsweise bei der optischen Aufhellung einer Reihe von Fasersubstanzen, z.B. von Polyesterfasern, mit den erfindungsgemässen Aufhellern zweckmässig in der Weise, dass man diese Fasern mit den wässrigen Dispersionen (gegebenenfalls auch Lösungen) der Aufhellmittel bei Temperaturen umter 75°C, z.B. bei Raumtemperatur, imprägniert und einer trockenen Wärmebehandlung bei Temperaturen über 100°C unterwirft, wobei es sich im allgemeinen empfielt, das Fasermaterial vorher noch bei mässig erhöhter Temperatur, z.B. bei mindestens 60°C bis etwa 130°C zu trocknen. Die Wärmebehandlung in trockenem Zustande erfolgt dann vorteilhaft bei Temperaturen zwischen 120 und 225°C, beispielsweise durch Erwärmen in einer Trockenkammer, durch Bügeln im angegebenen Temperaturintervall oder auch durch Behandeln mit trockenem, überhitztem Wasserdampf. Die Trocknung und trockene Wärmebehandlung können auch unmittelbar nacheinander ausgeführt oder in einen einzigen Arbeitsgang zusammengelegt werden.

Die Menge der erfindungsgemäss zu vervendenden neuen optischen Aufheller, bezogen auf das optisch aufzuhellende Material, kann in weiten Grenzen schwanken. Schon mit sehr geringen Mengen, in gewissen Fällen z.P. solchen von 0,0001 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,0005 und 0,5 Gewichtsprozent von Interesse.

Aus verschiedenen Gründen ist es oft zweckmässig, die Aufheller nicht als solche, d.h. rein einzusetzen, sondern vermischt mit den verschiedensten Hilfs- und Coupiermitteln, wie z.B. wasserfreiem Natriumsulfat, Natriumsulfat-decahydrat, Natriumchlorid, Natriumcarbonat, Alkalimetallphosphaten, wie Natrium- oder Kaliumorthophosphat, Natrium- oder Kaliumpyrophosphat und Natrium- oder- Kaliumtripolyphosphaten oder Alkalimetallsilicaten.

In den Beispielen sind Prozente immer Gewichtsprozente. Schmelz- und Siedepunkte sind, sofern nicht anders vermerkt, unkorrigiert.

**Beispiel 1:** 6,5 g des Aldehyds der Formel

(100) 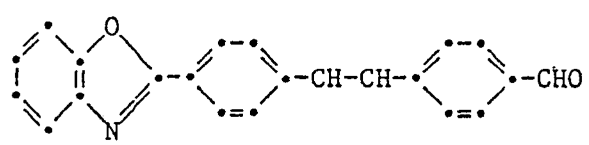

werden unter Rühren bei Raumtemperatur in 200 ml Aethylenglykolmonomethyläther suspendiert. 2,1 g O-Methyloydroxylaminhydrochlorid werden in 10 ml Wasser gelöst, zur Suspension des Aldehyds gegeben und dann 2,1 g Natriumacetat eingetragen, wobei die Temperatur, der Suspension von 25°C auf 28°C ansteigt. Es wird noch 6 Stunden bei Raumtemperatur nachgerührt, abgenutscht, mit Wasser gewaschen und bei 60°C unter Vakuum getrocknet. Nach zweimaliger Umkristallisation aus je 200 ml Chlorbenzol bzw. Toluol unter Zuhilfenahme von Bleicherde erhält man 3,9 g der Verbindung der Formel

(101) 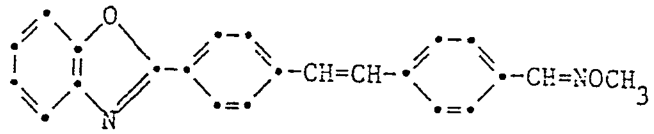

als hellgelbe Kristalle Schmelzpunkt: 247-248°C. Auf analoge Weise erhält man die Verbindungen der Formel

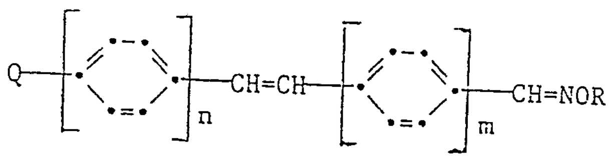

| Verbindung Nr. | Q | n | m | R | Schmelzpunkt °C |
|---|---|---|---|---|---|
| 102 | 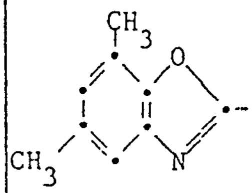 | 1 | 1 | $-CH_3$ | 165-166 |
| 103 | 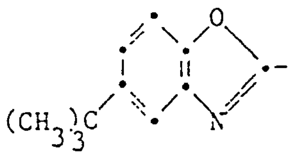 | 1 | 1 | $-CH_3$ | 239-240 |
| 104 | 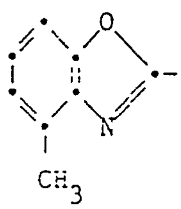 | 1 | 1 | $-CH_3$ | 191-193 |
| 105 | 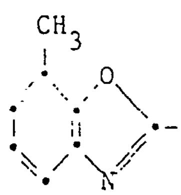 | 1 | 1 | $-CH_3$ | 166-168 |
| 106 | 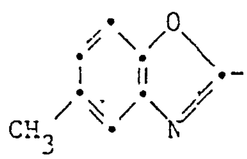 | 1 | 1 | $-CH_3$ | 285-286 |
| 107 | 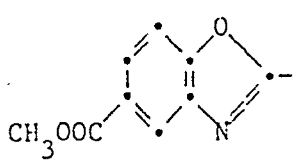 | 1 | 1 | $-CH_3$ | 271-272 |
| 108 | NC-CH=CH- | 1 | 1 | $-CH_3$ | 137-138 |

| Verbindung Nr. | Q | n | m | R | Schmelzpunkt °C |
|---|---|---|---|---|---|
| 109 | 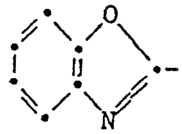 | 1 | 1 | $-C_2H_5$ | 187-188 |
| 110 | 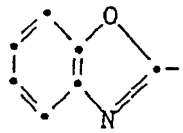 | 1 | 1 | $C_4H_9$(n) | 172-173 |
| 111 | 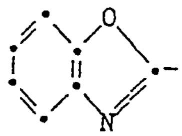 | 1 | 1 | $-CH(CH_3)_2$ | 253-254 |
| 112 | 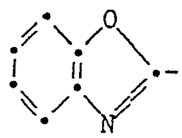 | 1 | 1 | 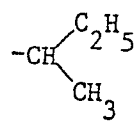 | 247-248 |
| 113 | 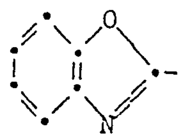 | 1 | 1 | $-CH_2-CH=CH_2$ | 181-182 |
| 114 | 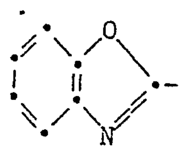 | 1 | 1 | $-CH_2CH_2OCH_3$ | 171-172 |
| 115 | 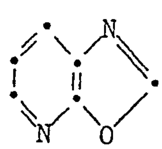 | 1 | 1 | $-C_2H_5$ | 255-256 |
| 116 | 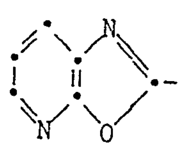 | 1 | 1 | $-C_4H_9$(n) | 273-274 |

| Verbindung Nr. | Q | n | m | R | Schmelzpunkt °C |
|---|---|---|---|---|---|
| 117 | 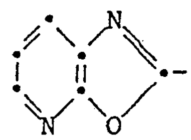 | 1 | 1 | $-CH(CH_3)_2$ | 267-268 |
| 118 | 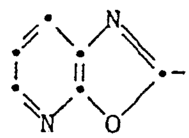 | 1 | 1 | $-CH\genfrac{}{}{0pt}{}{C_2H_5}{CH_3}$ | 260-261 |
| 119 | NC-CH=CH- | 1 | 1 | $-C_4H_9(n)$ | 118-119 |
| 120 | NC-CH=CH- | 1 | 1 | $-C_2H_5$ | 155-156 |
| 121 | NC-CH=CH- | 1 | 1 | $-CH(CH_3)_2$ | 140-141 |
| 122 | NC-CH=CH- | 1 | 1 | $-CH\genfrac{}{}{0pt}{}{C_2H_5}{CH_3}$ | 151-152 |
| 123 | NC-CH=CH- | 1 | 1 | $-CH_2CH_2OCH_3$ | 129-130 |
| 124 | NC-CH=CH- | 1 | 1 | $-CH_2-C\equiv CH$ | 158-159 |
| 125 | NC-CH=CH- | 1 | 1 | $-CH_2-CH=CH_2$ | 123-124 |
| 126 | 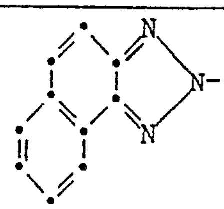 | 1 | 1 | $-CH_3$ | 188-189 |

| Verbindung Nr. | Q | n | m | R | Schmelzpunkt °C |
|---|---|---|---|---|---|
| 127 | 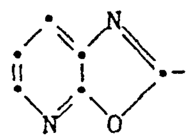 | 1 | 1 | $-CH_3$ | 237-238 |
| 128 | NC-CH=CH- | 1 | 1 | $-CH_2CH_2CN$ | 128-129 |
| 129 | NC-CH=CH- | 1 | 1 | $-CH_2COOC_2H_5$ | 131-132 |
| 130 | 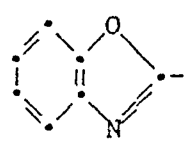 | 1 | 1 | $-CH_2CH_2CN$ | 253 Zers. |
| 131 | 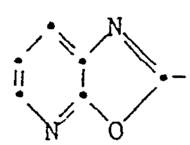 | 1 | 1 | $-CH_2CH_2CN$ | 277-278 |

**Herstellung des verwendeten Ausgangsproduktes der Formel (100)**

13,4 g Terephthalaldehyd werden bei Raumtemperatur unter Rühren in 60 ml Methanol suspendiert. Dann werden 36 g einer methanolischen Natrium-methylatlösung (Gehalt: 30 %) zugegeben. Es entsteht in einer exothermen Reaktion, wobei die Temperatur durch leichtes Kühlen bei 20-22°C gehalten wird, eine goldgelbe Lösung. Dann wird eine Lösung von 34,5 g des Phosphonates der Formel

(109) 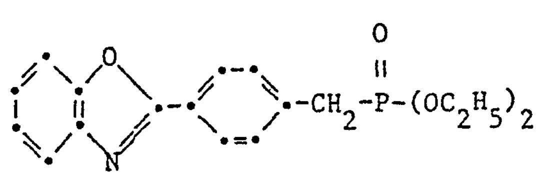

in 100 ml Methanol innert 20 Minuten zugetropft. Die entstandene Suspension wird noch 4 1/2 Stunden bei Raumtemperatur nachgerührt, das entstandene produkt abgenutscht, mit Methanol gut gewaschen und bei 60°C getrocknet. Man erhält so 20,0 g der Verbindung der Formel (100). Schmelzpunkt 231-232°C.

Auf analoge Weise erhält man die Aldehyde der Formel

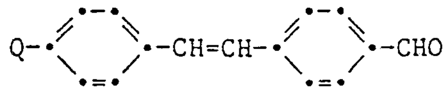

| Verbindung Nr. | Q | Schmelzpunkt °C |
|---|---|---|
| 132 | 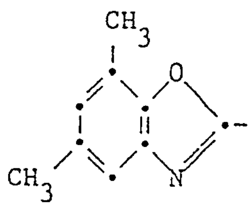 | 193-195 |
| 133 | 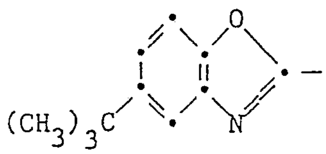 | 210-213 |
| 134 | 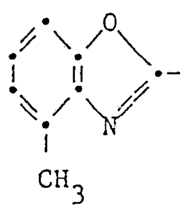 | 184-188 |

| Verbindung Nr. | Q | Schmelzpunkt °C |
|---|---|---|
| 135 | 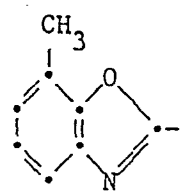 | 185-186 |
| 136 | 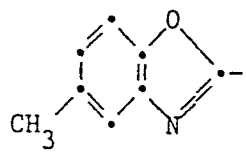 | 219-220 |
| 137 | 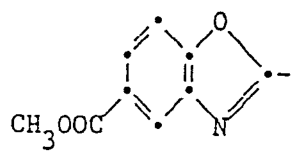 | 234-236 |
| 138 | NC-CH=CH- | 154-156 |
| 139 | 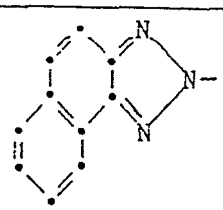 | 224-227 |
| 140 | 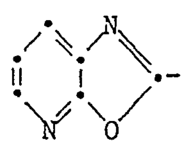 | 254-264 |

Die verwendeten Phosphonate sind bekannt oder können in an sich bekanter Weise wie beispielsweise in den DE-OS 28 16 511 oder 28 48 149 oder in der europäischen Patentanmeldung 31796 beschrieben, erhalten werden.

**Beispiel 2:**

3,8 g 2-[(4-Diäthoxyphosporylmethyl]-4-phenyl-1,3,4-oxdiazol-2-on (Vergleiche EP 9095) und 1,7 g des Aldehyds der Formel

(200) 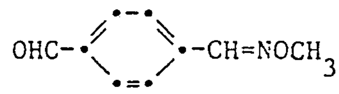

werden unter Rühren bei 40°C in 20ml Dimethylformamid gelöst. Dann werden 2 g einer methanolischen Natrium-methylatlösung (Gehalt. 30 %) zugegeben. Die rotbraune Lösung wird noch 2 Stunden bei 40°C gerührt, mit 40 ml Methanol versetzt auf 5°C abgekühlt, das auskristallisierte Produkt abgenutscht und unter Vakuum bei 70°C getrocknet. Nach Umkristallisation aus 100 ml Benzin vom Siedepunkt 110-150°C bzw. 100 ml Xylol unter Zuhilfenahme von Bleicherde erhält man 1,2 g der Verbindung der Formel

(201) 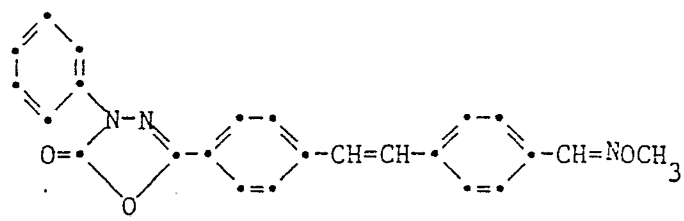

als gelbes Pulver. Schmelzpunkt: 165-186°C.

Der Aldehyd der Formel (200) kann wie folgt erhalten werden.

24 g p-Toluylaldehyd werden unter Rühren in 240 ml 2-Methoxy-äthanol bei Raumtemperatur gelöst. Dann werden je 20 ml einer wässrigen Lösung von 20,8 g 0-Methylhydroxylaminhydrochlorid bzw. 20,5 g-Natriumacetat zugegeben und anschliessend während 20 Stunden bei Raumtemperatur gerührt. Das Reaktionsgemisch wird in 600 ml Wasser gegossen, 400 ml Toluol zugegeben, gut durchgeschüttelt, die organische Phase abgetrennt, mit wasserfreiem Natriumsulfat getrocknet und das Lösungsmittel abdestilliert. Man erhält so 28,3 g der Verbindung der Formel

(202) 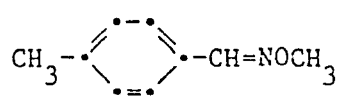

als blass-bräunliches Oel (Gehalt: 96,6 %)

28,3 g der Verbindung der Formel (202), 35,5 g N-Bromsuccinimid und 0,5 g Dibenzoylperoxid werden unter Rühren und unter Beleuchtung mit einer 500 Watt Halogenlampe in 430 ml Tetrachlorkohlenstoff langsam erhitzt. Bei 76°C setzt eine lebhafte Reaktion ein, welche etwa 2 Minuten dauert. Die Suspension wird noch 2 Stunden unter Rückfluss gekocht, auf Raumtemperatur abgekühlt und mit 500 ml Wasser ausgeschüttelt. Nach dem Trennen wird die organische Phase mit wasserfreiem Natriumsulfat getrocknet und das Lösungsmittel abdestilliert. Der Rückstand erstarrt zu einem Kristallbrei. Man erhält so 42 g der Verbindung der Formel

(203) 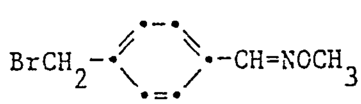 Schmelzpunkt 70-71°C .

In 500 ml Methanol werden unter Rühren bei Raumtemperatur 34,7 g 2-Nitropropan und 54 g methanolischen Natrium-methylatlösung (Gehalt: 30 %) eingetropft und eine Stunde gerührt. Dann werden 68,4 g der Verbindung (203) eingetragen, auf 45°C erwärmt und eine Stunde bei dieser Temperatur und ca. 20 Stunden bei Raumtemperatur gerührt.

Nach dem Eindampfen wir das Produkt durch Säulenchromatographie mittels Kieselgel gereinigt. Eluiermittel:Toluol. Man erhält so 31,2 g des Aldehyds der Formel (200). Schmelzpunkt: 66-67°C.

Auf analoge Weise wie am Anfang dieses Beispiels beschrieben erhält man die Verbindungen der Formel

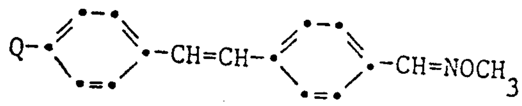

| Verb.Nr. | Q | Schmelzpunkt (°C) |
|---|---|---|
| 204 | 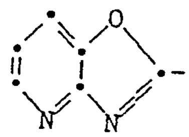 | 236-237 |
| 205 | 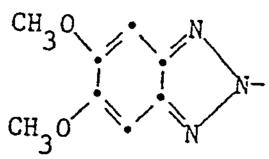 | 271-273 |
| 206 | 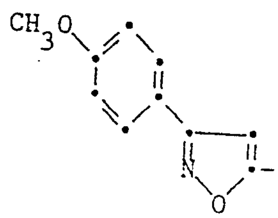 | 212-213 |
| 207 | 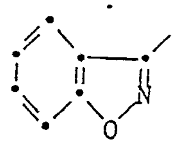 | 181-182 |
| 208 | 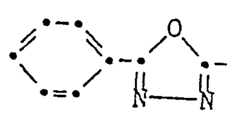 | 169-170 |
| 209 | 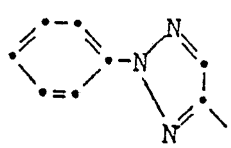 | 196-197 |

| Verb.Nr. | Q | Schmelzpunkt (°C) |
|---|---|---|
| 210 | 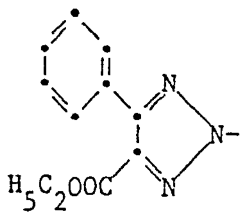 | 155-156 |
| 211 | 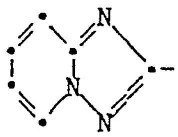 | 234-235 |
| 212 | 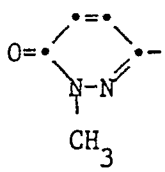 | 207-208 |
| 213 | 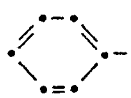 | 244-245 |

**Beispiel 3:**

4,7 g Stilben-4,4'-dialdehyd werden unter Rühren bei 60° C in 200 ml 2-Methoxy-äthanol gelöst, dann werden je 10 ml einer wässrigen Lösung von 5 g O-Methylhydroxylamin-hydrochlorid bzw. 5 g Natriumacetat zugegeben, noch eine Stunde bei 60°C und ca. 20 Stunden bei Raumtemperatur gerührt. Das auskristallisierte Produkt wird abgenutscht, viermal aus 2-Methoxy-äthanol und 2mal aus Xylol unter Zuhilfenahme von Bleicherde umkristallisiert. Man erhält so 0,4 g der Verbindung der Formel

(300) 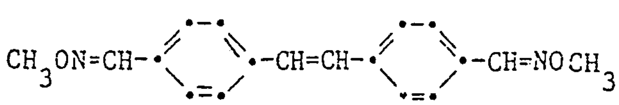

Grünstichige Nädelchen. Schmelzpunkt: 190-191°C

**Beispiel 4:**

6,7 g des Aldehyds der Formel

(400) 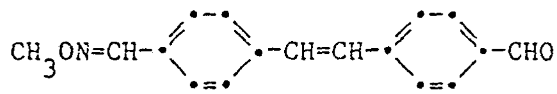

und 4,8 g des Phosphonates der Formel

(401) 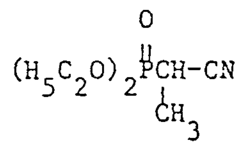

werden unter Rühren in einem Gemisch von 200 ml Dimethylformamid und 100 ml N-Methylpyrrolidon bei 65° C gelöst. Innert 10 Miunten werden dann 5 4 g einer methanolischen Natrium-methylatlösung (Gehalt. 30 %) zugetropft und während ca. 20 Stunden bei Raumtemperatur gerührt. Nach dem Klärfiltrieren wird das Filtrat mit 300 ml Wasser versetzt, das ausgefallene Produkt abgenutscht, mit 200 ml Methanol verrührt, wieder abgenutscht und zweimal aus 70 ml Toluol unter Zuhilfenahme von Bleicherde umkristallisiert. Man erhält so das Produkt der Formel

(402) 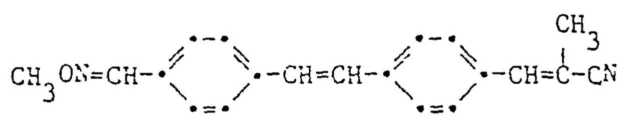

als hellgelbe Kristalle vom Schmelzpunkt 162-163° C.

Verwendet man anstelle des Phosphonates der Formel (401) dasjenige der Formel

(403) 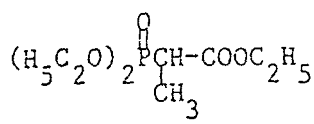

erhält man unter Umesterung die Verbindung der Formel

(404) 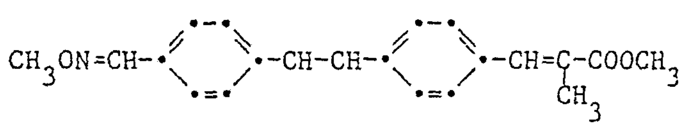

Schmelzpunkt: 163-164° C.

Der Aldehyd der Formel (400) kann wie folgt erhalten werden:

42 g der Verbindung der Formel (203) werden unter Rühren zusammen mit 91 g Trimethylphosphit auf ca. 110° C erhitzt und während etwa 20 Stunden bei dieser Temperatur gerührt. Dann wird das überschüssige Trimethylphosphit unter Wasserstrahlvakuum abdestilliert und der Rückstand durch Säulenchromatographie über Kieselgel gereinigt. Eluiermittel: Zuerst Toluol/Essigester 9:1 dann Essigester. Man erhält so 35,5 g des Phosphonats der Formel

(405) 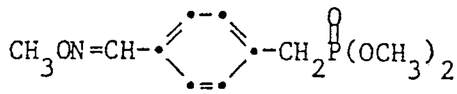

Goldgelbes Oel.

13,4 g Terephthalaldehyd werden unter Rühren bei Raumtemperatur in 60 ml Methanol suspendiert. Nach Zugabe von 18 g einer methanolischen Lösung von Natrium-methylat (Gehalt: 30 %) entsteht eine gelbe Lösung. Innert einer Stunde werden dann 25,7 g des Phosphonates der Formel (405) gelöst in 60 ml Methanol bei 18 - 20°C zugetropft und 20 Stunden bei Raumtemperatur nachgerührt. Das auskristallisierte Produkt wird abgenutscht, mit Methanol gewaschen und unter Vakuum getrocknet. Man erhält so 12,8 g der Verbindung der Formel (400). Schmelzpunkt: 115-118°C.

**Beispiel 5:**

5,1 g des Phosphonates der Formel (405) und 3 9 g 4-Formylzimtsäure-methylester werden unter Rühren bei 40°C in 60 ml Dimethylformamid gelöst. Nach Zugabe von 4 g einer methanolischen Natrium-methylatlösung (Gehalt: 30 %) wird noch 2 Stunden bei 40°C nachgerührt. Das Reaktionsgemisch wird in 240 ml Methanol gegossen, das auskristallisierte Produkt abgenutscht, mit Methanol gewaschen und unter Vakuum bei 80°' getrocknet. Nach dreimaligem Umkristallisieren aus Toluol unter Zuhilfenahme von Bleicherde erhält man 2,3 g der Verbindung der Formel

(500) 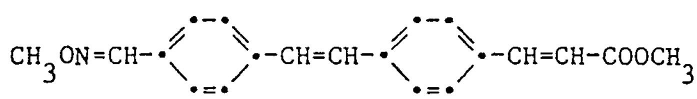

Hellgelbe Nadeln. Schmelzpunkt: 213-214°C.

Verwendet man anstelle des Methylesters die äquivalente Menge 4-Formylzimtsäure-äthylester und als Kondensationsmittel Natriumäthylat, erhält man die Verbindung der Formel

(501) 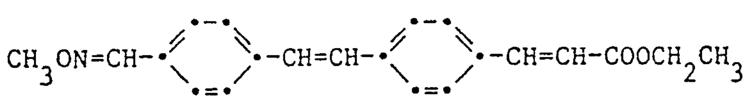

Blassgelbe Kristalle. Schmelzpunkt: 209-210°C.

**Beispiel 6:** 6,8 g des Aldehyds der Formel

(600) 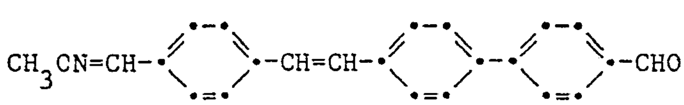

und 3,5 g des Phosphonats der Formel

(601) $(H_5C_2O)_2\overset{O}{\overset{\|}{P}}CH_2-CN$

werden unter Rühre i 150 ml N-Methylpyrrolidon bei 50°C gelöst. Nach Zugabe von 4 g einer methanolischen Natrium-methylatlösung (Gehalt: 30 %) wird noch ca. 20 Stunden bei Raumtemperatur nachgerührt. Die klärfiltrierte Lösung wird mit 300 ml Methanol verdünnt, das auskristallisierte Produkt abgenutscht, mit Methanol gewaschen und 2mal aus je 400 ml Chlorbenzol unter Zuhilfenahme von Bleicherde umkristallisiert. Man erhält so 2,5 g der Verbindung der Formel

(602) 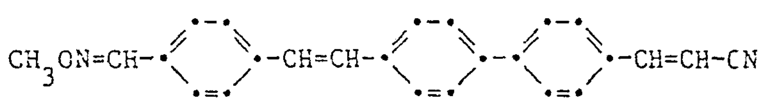

blassgelbe Kristalle. Schmelzpunkt: 246 - 247°C.

Der Aldehyd der Formel (600) kann wie folgt erhalten werden:

12,6 g Biphenyl-4,4'-dialdehyd und 11 g Natriummethylat werden bei Raumtemperatur unter Rühren in 40 ml Methanol suspendiert. Das Phosphonat der Formel (405) wird in 40 ml Methanol gelöst und innert 40 Minuten zugetropft. Nach ca. 20 stündigem Nachrühren wird das Produkt abgenutscht, mit viel Methanol gewaschen und unter Vakuum bei 80°C getrocknet. Man erhält so 12,5 g des Aldehyds der Formel (600) Schmelzpunkt: 134-136°C.

**Beispiel 7:** 6,7 g des Aldehyds der Formel

(700) 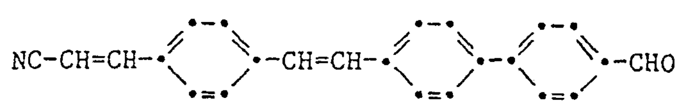

werden unter Rühren bei 70°C in 300 ml 2-Methoxy-äthanol gelöst. 2,5 g O-Methylhydroxylamin-hydrochlorid und 2,5 g Natriumacetat werden in je 5 ml Wasser geloöst und zugegeben. Dann wird 6 Stunden bei Raumtemperatur nachgerüht das auskristallisierte Produkt abgenutscht und nach dem Trocknen aus 70 ml Chlorbenzol unter Zuhilfenahme von Bleicherde umkristallisiert. Man erhält so 1,5 g der Verbindung der Formel

(701) 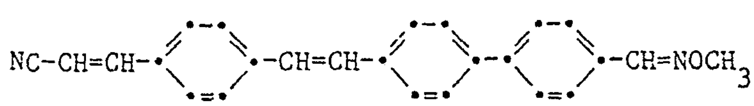

Hellgelbe Kristalle. Schmelzpunkt: 236-237°C.

Der Aldehyd der Formel (700) kann wie folgt erhalten werden:

8,4 g Biphenyl-4,4'-dialdehyd und 7,2 g einer methanolischen Natriummethylatlösung (Gehalt: 30 %) werden unter Rühren in 60 ml Methanol vorgelegt. 10 g 4-(Dimethoxyphosphorylmethylphenyl)-zimtsäurenitril werden in 60 ml Methanol gelöst und innert 40 Minuten bei Raumtemeratur zugetropft. Es wird noch 20 Stunden bei Raumtemperatur gerührt, auf 5°C abgekühlt, das auskristallisierte Produkt abgenutscht mit viel Methanol gewaschen und unter Vakuum getrocknet. Man erhält so 6,7 g der Verbindung der Formel (700) Schmelzpunkt:. 127-131°C.4-(Dimethoxyphosphorylmethylphenyl)-zimtsäurenitril kann wie folgt erhalten werden: 390 g 4-Brommethylzimtsäurenitril werden in 600 ml Trimethylphosphit suspendiert und zu 250 ml auf 90°C erhitztes und gut gerührtes Trimethylphosphit portionenweise so zudosiert, dass die stark exotherme Reaktion unter Kontrolle gehalten werden kann. Dann wird innert 2 Stunden langsam auf 110°C erhitzt. Das überschüssige

Trimethylphosphit wird dann unter Wasserstrahlvakuum abdestilliert, zum Rückstand 330 ml Toluol und 660 ml Hexan zugegeben, auf 0°C abgekühlt, das auskristallisierte Produkt abgenutscht und unter Vakuum bei 50°C getrocknet.Man erhält so 427 g 4-(Dimethoxyphosphoryl-methylphenyl)-zimtsäurenitril vom Schmelzpumkt 98-101°C.

**Beispiel 8:**

5 g 4-(Dimethoxyphosphorylmethylphenyl)-zimtsäurenitril und 3,6 g des Aldehyds der Formel

(800) 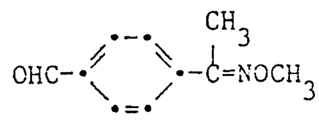

werden in 50 ml Dimethylformamid unter Rühren gelöst. Dann werden 4 g einer methanolischen Natrium-methylatlösung (Gehalt: 30 %) innert 15 Minuten zugetropft. Es wird noch 2 Stunden bei 40°C nachgerührt, 250 ml Methanol zugegeben, auf 5°C abgekühlt, das auskristallisierte Produkt abgenutscht und unter Vakuum getrocknet. Nach Umkristallisation aus Toluol unter Zuhilfenahme von Bleicherde erhält man 2,7 g der Verbindung der Formel

(801) 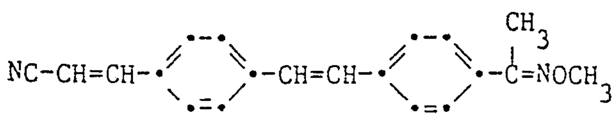

Hellgelbe Blättchen. Schmelzpunkt: 168-169°C.

Auf analoge Weise können die Verbindungen der Formeln

(802) 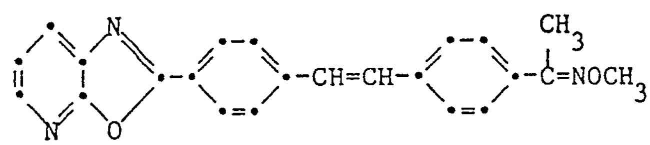

Blassgelbe Blättchen. Schmelzpunkt: 245-246 und

(803) 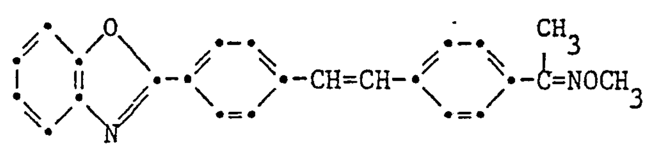

Blassgelbe Blättchen. Schmelzpunkt: 239 - 240°C erhalten werden.

Der Aldehyd der Formel (800) kann wie folgt erhalten werden

134 g 4-Methyl-acetophenon werden unter Rühren bei Raumtemperatur in 2000 ml 2-Methoxy-äthanol vorgelegt. Dann werden je 200 ml einer Lösung von 100 g O-Methylhydroxylamin-hydrochlorid und 98 g Natriumacetat in Wasser zugegeben. Es wird 24 Stunden bei Raumtemperatur nachgerührt, das Reaktionsgemisch in 8000 ml Wasser gegossen und mit 200 ml Aether extrahiert. Die Aetherphase wird abgetrennt über wasserfreiem Natriumsulfat getrocknet und eingedampft. Man erhält so 153,2 g der Verbindung der Formel

(804) 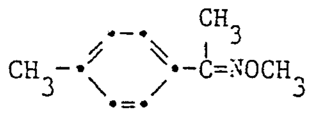

als blassgelbe Flüssigkeit.

153 g der Verbindung der Formel (804), 178 g N-Bromsuccinimid und 5 g Dibenzoylperoxid werden unter Rühren und unter Beleuchtung mit einer 500 Watt Halogenlampe in 1500 ml Tetrachlorkohlenstoff langsam zum Rückfluss erhitzt und während 6 Stunden unter Rückfluss gekocht. Dann wird heiss abgenutscht, das abgekühlte Filtrat mit 1500 ml Wasser ausgeschüttelt, über wasserfreiem Natriumsulfat getrocknet und eingedampft. Nach Reinigung über eine Kieselgelsäule (Eluiermittel: Toluol) erhält man 190 g der Verbindung der Formel

(805) 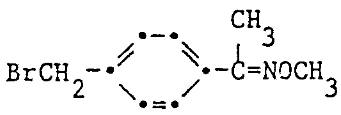

als gelbe Flüssigkeit. Gehalt: 58 % (GC).

141 g einer methanolischen Natrium-methylatlösung (Gehalt: 30 %) werden unter Rühren in 1500 ml absolutem Aethanol vorgelegt. Dann werden 89 g 2-Nitropropan zugetropft und während einer Stunde bei Raumtemperatur gerührt. Nach dem Eintragen von 190 g der Verbindung der Formel (805) steigt die Temperatur auf 36°C an. Es wird noch 4 Stunden bei Raumtemperatur gerührt, eingedampft und über eine Kieselgelsäule gereinigt. Eluiermittel: Zuerst Hexan-Toluol 1:1 dann Toluol. Man erhält so 70 g des Aldehyds der Formel (800) als blassbraunes Oel. Gehalt: 92,5 % (GC)

**Beispiel 9:**

Man foulardiert bei Raumtemperatur ein Polyestergewebe (Terylen® Typ 540) mit einer Flotte, welche pro Liter 1 g der Verbindung der Formel (101), (102), (103) oder (108) und 1 ml des Kondensationsproduktes von 8 - 9 Mol Aethylenoxid mit 1 Mol p-tert.-Octylphenol enthält. Der Abquetscheffekt beträgt 80 %. Anschliessend wird 10 Minuten bei 80°C getrocknet und dann 30 Sekunden bei 220°C thermofixiert. Das so behandelte Gewebe weist einen hohen Aufhelleffekt auf.

**Beispiel 10:**

Man foulardiert bei Raumtemperatur ein Polyestergewebe (Terylen® Typ 540) mit einer Flotte, welche pro Liter 1 g der Verbindung der Formel (108) und 1 ml des Kondensationsproduktes von 8-9 Mol Aethylenoxid mit 1 Mol p-tert.-Octylphenol enthält. Der Abquetscheffekt beträgt 80 %. Anschliessend wird 10 Minuten bei 80°C getrocknet und dann 30 Sekunden bei 200°C thermofixiert. Das so behandelte Gewebe weist einen hohen Aufhelleffekt auf.

**Beispiel 11:**

Ein Polyestergewebe (Terylen® Typ 540) wird auf ein Färbeapparat bei einem Flottenverhältnis von 1:20 mit einem wässrigen Bad behandelt, welches 0,1 %, (bezogen auf das Warengewicht) der Verbindung der Formel (101), (102), (104), (106) oder (108) und 1 g/Liter des Kondensationsproduktes von 35 Mol Aethylenoxid mit 1 Mol Stearylalkohol enthält. Man erwärmt nun das Bad innerhalb 30 Minuten von 40 auf 120°C, behält es während 30 Minuten auf dieser Temperatur und kühlt es dann innerhalb 15 Minuten auf 15°C ab. Das Gewebe wird anschliessend in fliessendem deionisiertem Wasser gespürt und bei 70°C getrocknet. Das so behandelte Polyestergewebe weist einen hohen Aufhelleffekt auf.

**Beispiel 12:**

Ein Polyamid-6,6-Webtricot wird auf einem Färbeapparat bei einem Flottenverhältnis von 1:20 mit einem wässrigen Bad behandelt, welches 0,2 %, bezogen auf das Warengewicht, der Verbindung (101) (102), (104) oder (106)und 3 g/Liter eines Gemisches von 60 Gewichtsteile Natriumhydrosulfit und 40 Gewichtsteile Natriumpyrophosphat enthält. Man erwärmt das Bad innerhalb 30 Minuten von 40 auf 130° C, behält es während 30 Minuten auf dieser Temperatur und kühlt dann innerhalb 15 Minuten auf 40° C ab. Das Gewebe wird anschliessend in fliessendem deionisiertem Wasser gespült und mit einem 180° C warmen Bügeleisen getrocknet Das so behandelte Polyamidgewebe weist einen hohen Aufhelleffekt auf.

**Patentansprüche:**

1. Stilbenverbindungen der Formel

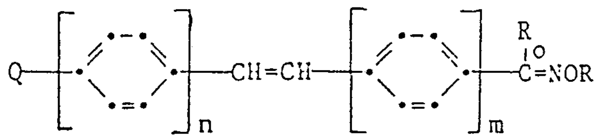

,

worin Q Wassersdoff; einen monocyclischen 5- oder 6-gliedrigen unsubstituierten oder nicht-chromophor substituierten 0, 1 oder 2 ankondensierte Benzolringe aufweisenden aromatischen heterocyclischen Ring oder den Rest -CH=C($R_1$)($R_2$), worin $R_1$ für Wasserstoff, unsubstituiertes oder nicht-chromophor substituiertes $C_{1-6}$ -Alkyl und $R_2$ für Alkoxycarbonyl mit insgesamt 2 bis 7 C-Atomen, Cyano, Carboxy, Carbrmoyl, Phosphonsäuredialkylester, $C_{1-6}$ -Alkyl- oder Arylsulfonyl

stehen oder Q auch 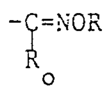 bedeuten kann, $R_O$ Wasserstoff oder

,

$C_{1-6}$ -Alkyl, R unsubstituiertes oder nicht-chromophor substituiertes $C_{1-6}$ -Alkyl, $C_{2-5}$ -Alkenyl, $C_{2-5}$ -Alkinyl, Di-$C_{1-4}$ -alkylamino-$C_{14}$ -alkyl, Hydroxy-$C_{1-4}$ -alkyl, Phenyl-$C_{1-4}$ -alkyl oder Cycloalkyl mit 5 bis 12 Ringgliedern und m und n unabhängig voneinander die Zahl 1 oder 2 bedeuten.

2. Stilbenverbindungen gemäss Anspruch 1 der Formel

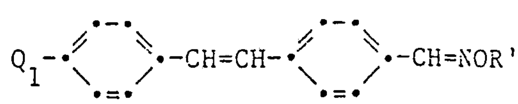

,

R' $C_{1-4}$ -Alkyl, Cyano-$C_{1-4}$ -Alkyl, $C_{1-4}$ -Alkoxy- $C_{2-4}$ -Alkyl, $C_{3-4}$-Alkenyl, $C_{3-4}$ -Alkinyl oder $R_x$ OOC-$C_{1-4}$- Alkyl, worin $R_x$ für Wasserstoff oder $C_{1-4}$ -Alkyl steht und $Q_1$ Phenyl oder den Rest -CH=C($R_1$')($R_2$'),

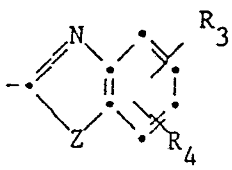 , 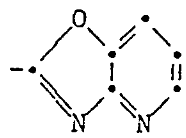 , 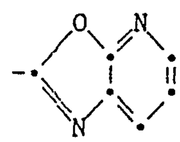 ,

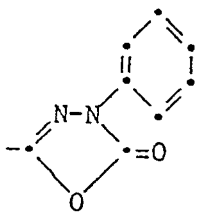 , 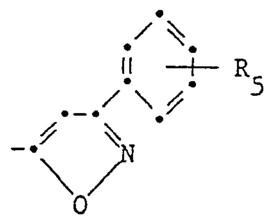 , 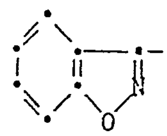 , 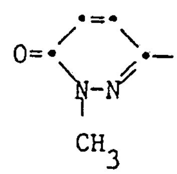 ,

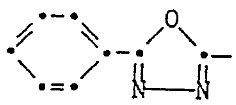 , 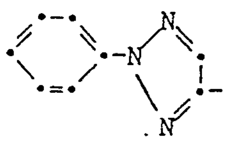 , 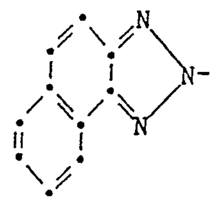

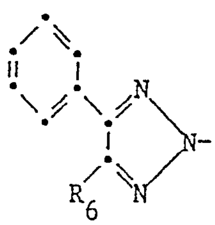 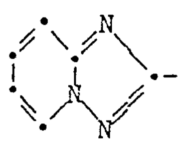 oder 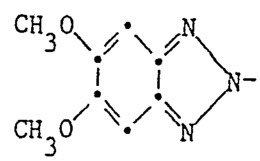

bedeuten, worin R'$_1$ Wasserstoff oder $C_{1\text{-}4}$ -Alkyl, R'$_2$ Alkoxycarbonyl mit insgesamt 2 bis 5 C-Atomen, Cyano, Carboxy, Carbamoyl, $C_{1\text{-}4}$ -Alkylsulfonyl oder Phenylsulfonyl, $R_3$ Wasserstoff, Chlor, $C_{1\text{-}4}$ -Alkyl, Phenyl-$C_{1\text{-}3}$ -alkyl Cyclohexyl, Phenyl, $C_{1\text{-}4}$ -Alkoxy, $C_{1\text{-}4}$ -Alkylsulfonyl, $C_{2\text{-}5}$ -Alkoxycarbonyl, Cyano oder Carboxy oder zusammen mit $R_4$ einen unsubstituierten oder durch 1 bis 4 Methylgruppen substituierten ankondensierten Cyclopentan-, Cyclohexan- oder Benzolring, $R_4$ Wasserstoff, Chlor, $C_{1\text{-}4}$ -Alkyl oder $C_{1\text{-}4}$ -Alkoxy oder zusammen mit $R_3$ einen unsubstituierten oder durch 1 bis 4 Methylgruppen substituierten ankondensierten Cyclopentan- Cyclohexan- oder Benzolring $R_5$ Wasserstoff oder Methoxy, $R_6$ Wasserstoff, $C_{1\text{-}4}$ -Alkyl oder Alkoxycarbonyl mit insgesamt 2 bis 5 C-Atomen und Z O, S oder NX bedeuten, worin X für Wasserstoff, $C_{1\text{-}4}$ -Alkyl oder Phenyl steht.

3. Stilbenverbindungen gemäss Anspruch 2 der Formel

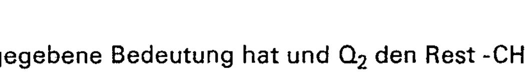

worin R' die in Anspruch 2 angegebene Bedeutung hat und $Q_2$ den Rest -CH=C($R_1$')($R_2$''),

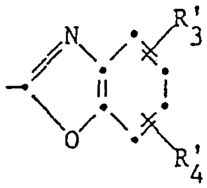, 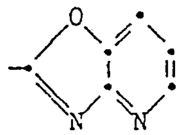 oder

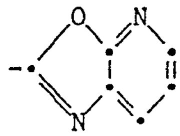

bedeutet, worin $R_1'$ Wasserstoff oder $C_{1-4}$-Alkyl, $R_2''$-Alkoxycarbonyl mit insgesamt 2 bis 5 C-Atomen oder Cyano, $R_3'$ Wasserstoff, $C_{1-4}$-Alkyl, $C_{1-4}$-Alkoxy oder Alkoxycarbonyl mit insgesamt 2 bis 5 C-Atomen oder zusammen mit $R_4'$ einen ankondensierten Benzolring und $R_4'$ Wasserstoff oder $C_{1-4}$-Alkyl oder zusammen mit $R_3'$ einen ankondensierten Benzolring bedeuten.

4. Stilbenverbindungen gemäss Anspruch 3 der Formel

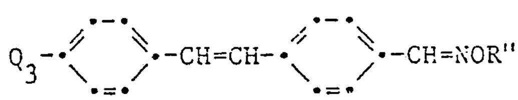 ,

worin R'' $C_{1-4}$-Alkyl, $C_{1-4}$-Alkoxy-$C_{2-4}$-alkyl oder $C_{3-4}$-Alkenyl und $Q_3$ den Rest -CH=CH-CN, -CH=CH-COO$C_{1-4}$-Alkyl oder den Rest

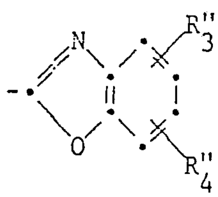

bedeuten, worin $R_3''$ für Wasserstoff, $C_{1-4}$-Alkyl, $C_{1-4}$-Alkoxy oder $C_{2-5}$-Alkoxycarbonyl und $R_4''$ für Wasserstoff oder $C_{1-4}$-Alkyl stehen.

5. Stilbenverbindungen gemäss Anspruch 4 der Formel

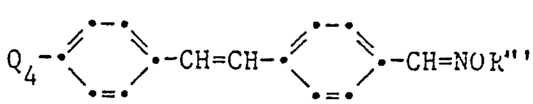 ,

worin $R_3'''$ $-CH_3$, $-C_2H_5$, $-CH_2CH_2OCH_3$ oder $-CH_2-CH=CH_2$ und $Q_4$ den Rest -CH=CH-CN, -CH=CH-COO$C_{1-2}$-Alkyl oder

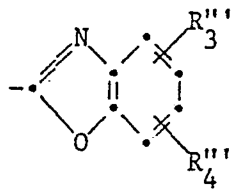

bedeutet, worin $R_3'''$ Wasserstoff oder $C_{1-4}$-Alkyl und $R_4'''$ Wasserstoff oder $C_{1-4}$-Alkyl bedeuten.

6. Die Stilbenverbindung gemäss Anspruch 5 der Formel

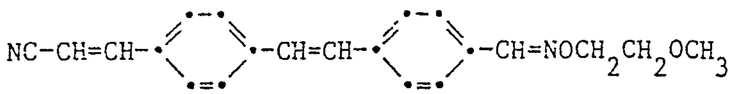 .

7. Die Stilbenverbindung gemäss Anspruch 5 der Formel

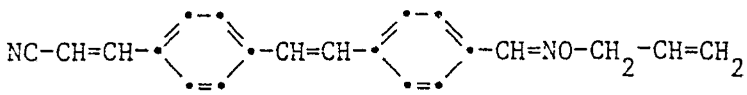

8. Verfahren zur Herstellung von Stiblenverbindungen der Formel

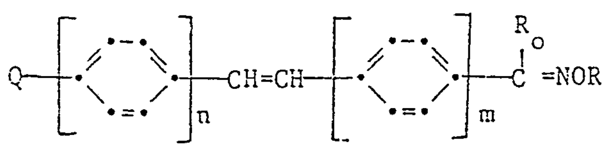 ,

worin Q, R, $R_o$, m und n die im Anspruch 1 angegebene Bedeutung haben, dadurch gekennzeichnet, dass man eine Carbonylverbindung der Formel

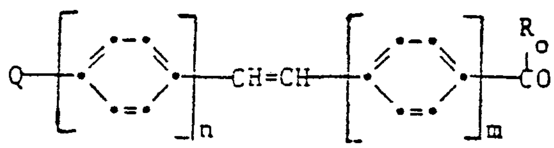

mit einem O-substituierten Hydroxylamin der Formel
$H_2NOR$
in einem inerten organischen Lösungsmittel umsetzt.

9. Verfahren zur Hertstellung von Stilbenverbindungen der Formel

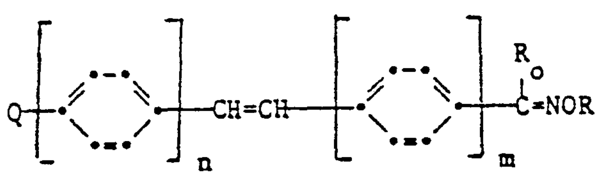 ,

worin Q, R, $R_o$, m und n die im Anspruch 1 angegebene Bedeutung haben, dadurch gekennzeichnet, dass man ein Mol einer Verbindung der Formel

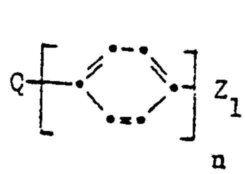

mit einem Mol einer Verbindung der Formel

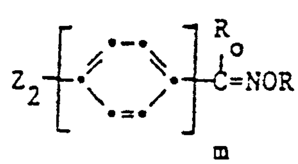

in Anwesenheit einer starken Base und eines polaren Lösungsmittels umsetzt, wobei eines der Symbole $Z_1$ und $Z_2$ eine HOC-Gruppe und das andere eine Gruppierung der Formel

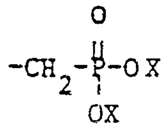, 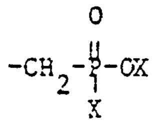, 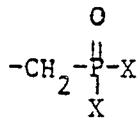 oder 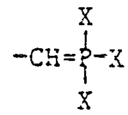

bedeuten, worin X einen unsubstituierten oder substituierten Alkylrest mit 1 bis 6 C-Atomen, einen Arylrest, einen Cycloalkylrest oder einen Aralkylrest darstellt.

10. Verfahren zum optischen Aufhellen von hochmolekularen synthetischen, halbsynthetischen und natürlichen organischen Materialien, dadurch gekennzeichnet, dass man Stilbenverbindungen, wie in einem der Anderen Oberfläche aufbringt.

11. Verfahren gemäss Anspruch 10 zum optischen Aufhellen von Polyestern nach dem Foulardtherm- und dem Ausziehverfahren.

12. Aufhellermischung enthaltend eine Stilbenverbindung gemäss den Ansprüchen 1 bis 6 und einen an sich bekannten optischen Aufheller im Verhältnis 1:9 bis 9:1.

13. Aufhellermischung gemäss Anspruch 12 enthaltend den Aufheller der Formel

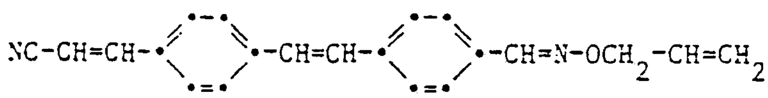

als Stilbenverbindung.

14. Aufhellermichung gemäss Anspruch 13 enthaltend den Aufheller der Formel

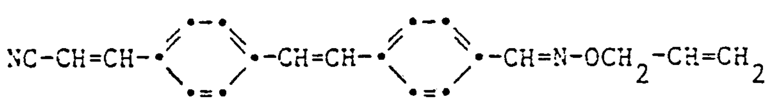

und den Aufheller der Formel

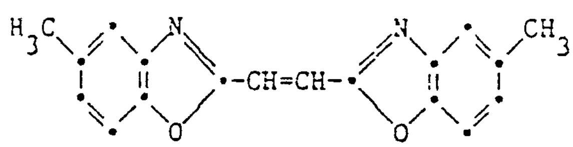

im Verhältnis 1:9 bis 9:1

**Claims:**

1. A stilbene compound of the formula

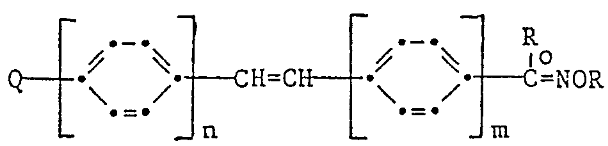 ,

wherein Q is hydrogen; a monocyclic 5- or 8-membered aromatic heterocyclic ring which is unsubstituted or substituted by non-chromophoric groups and which contains no fused benzene rings or one or two fused benzene rings; a bicyclic 9-membered aromatic heterocyclic ring or the radical -CH=C($R_1$)($R_2$), in which $R_1$ is hydrogen, $C_1$-$C_6$ alkyl which is unsubstituted or substituted by non-chromophoric groups, and $R_2$ is alkoxycarbonyl containing altogether 2 to 7 carbon atoms, cyano, carboxyl, carbamoyl, phosphonic

acid dialkyl ester, $C_1$-$C_6$alkyl or arylsulfonyl; or Q may also be $-\underset{R_o}{C}=NOR$, $R_o$ is hydrogen or $C_1$-$C_6$alkyl, R is

$C_1$-$C_6$ alkyl which is unsubstituted or substituted by non-chromophoric groups, or is $C_2$-$C_5$ alkenyl, $C_2$-$C_5$ alkynyl, di($C_1$-$C_4$)alkylamino($C_1$-$C_4$)alkyl, $C_1$-$C_4$ hydroxyalkyl, $C_1$-$C_4$ phenylalkyl or cycloalkyl containing 5 to 12 ring members,and each of m and n independently of the other is 1 or 2.

2. A stilbene compound according to claim 1 of the formula

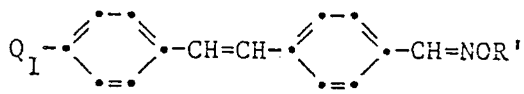 ,

wherein R′ is $C_1$-$C_4$alkyl, $C_1$-$C_4$ cyanoalkyl, $C_1$-$C_4$alkoxy-$C_2$-$C_4$alkyl, $C_3$-$C_4$alkenyl, $C_3$-$C_4$alkynyl or $R_xOOC$-$C_1$-$C_4$alkyl, in which $R_x$ is hydrogen or $C_1$-$C_4$alkyl, and $Q_1$ is phenyl or the radical $-CH=C(R_1')(R_2')$,

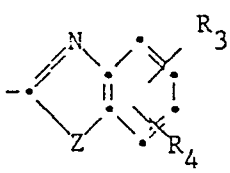 , 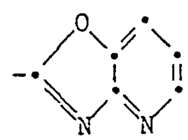 , 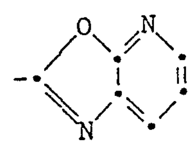 ,

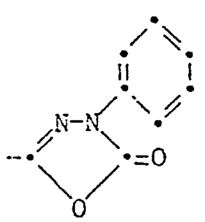 , 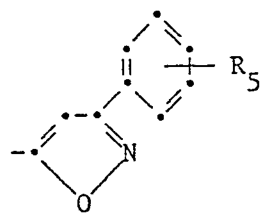 , 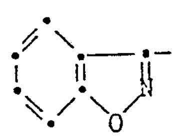 , 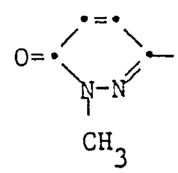 ,

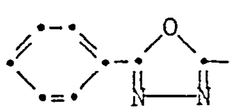 , 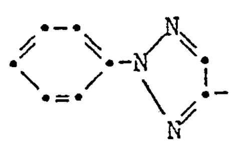 , 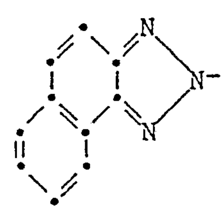

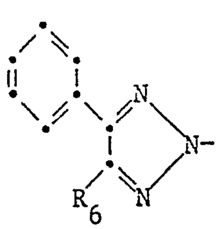 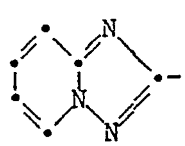 or 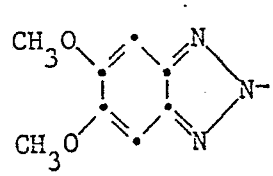

wherein $R_1'$ is hydrogen or $C_1$-$C_4$alkyl, $R_2'$ is alkoxycarbonyl containing altogether 2 to 5 carbon atoms, cyano, carboxyl, carbamoyl, $C_1$-$C_4$alkylsulfonyl or phenylsulfonyl, $R_3$ is hydrogen, chlorine, $C_1$-$C_4$alkyl, $C_1$-$C_3$phenylalkyl, cyklohexyl, phenyl, $C_1$-$C_4$alkoxy, $C_1$-$C_4$alkylsulfonyl, $C_{2i}$-$C_5$alkoxycarbonyl, cyano or carboxyl or, together with $R_4$,

is a fused cyclopentane, cyclohexane or benzene ring, each of which is unsubstituted or substituted by 1 to 4 methyl groups; $R_4$ is hydrogen, chlorine, $C_1$-$C_4$alkyl or $C_1$-$C_4$alkoxy or, together with $R_3$, is a fused cyclopentane, cyclohexane or benzene ring, each of which is unsubstituted or substituted by 1 to 4 methyl groups; $R_5$ is hydrogen or methoxy; $R_6$ is hydrogen, $C_1$-$C_4$alkyl or alkoxycarbonyl containing altogether 2 to 5 carbon atoms; and Z is O, S or NX, wherein X is hydrogen, $C_1$-$C_4$alkyl or phenyl

3. A stilbene compound according to claim 2 of the formula

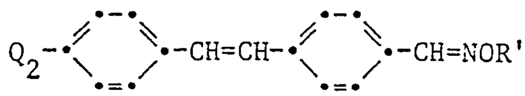

wherein R' is as defined in claim 2 and $Q_2$ is the radical $-CH=C(R_1')(R_2'')$,

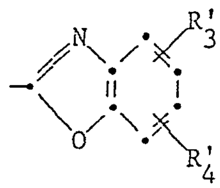

,

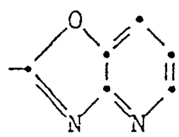

or

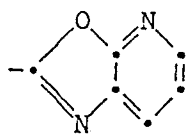

wherein $R_1'$ is hydrogen or $C_1$-$C_4$alkyl, $R_2''$ is alkoxycarbonyl containing altogether 2 to 5 carbon atoms or is cyano, $R_3'$ is hydrogen, $C_1$-$C_4$alkyl, $C_1$-$C_4$alkoxy or alkoxycarbonyl containing altogether 2 to 5 carbon atoms or, together with $R_4'$, is a fused benzene ring, and $R_4'$ is hydrogen or $C_1$-$C_4$alkyl or together with $R_3'$ is a fused benzene ring.

4. A stilbene compound according to claim 3 of the formula

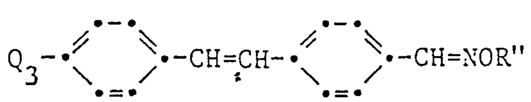

,

wherein R'' is $C_1$-$C_4$alkyl, $C_1$-$C_4$alkoxy-$C_2$-$C_4$alkyl or $C_3$-$C_4$alkenyl, and $Q_3$ is the radical $-CH=CH-CN$, $-CH=CH-COO-C_1$-$C_4$alkyl or the radical

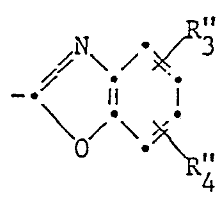

wherein $R_3''$ is hydrogen, $C_1$-$C_4$alkyl, $C_1$-$C_4$alkoxy or $C_2$-$C_5$alkoxycarbonyl, and $R_4''$ is hydrogen or $C_1$-$C_4$alkyl.

5. A stilbene compound according to claim 4 of the formula

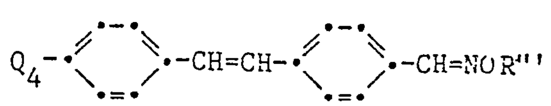

,

wherein $R_3'''$ is $-CH_3$, $-C_2H_5$, $-CH_2CH_2OCH_3$ or $-CH_2-CH=CH_2$ and $Q_4$ is the radical $-CH=CH-CN$, $-CH=CH-COO-C_1$-$C_2$alkyl or

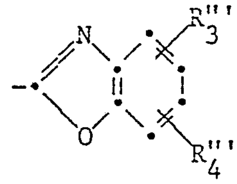

wherein $R_3'''$ is hydrogen or $C_1$-$C_4$alkyl and $R_4'''$ is hydrogen or $C_1$-$C_4$alkyl.

6. The stilbene compound according to claim 5 of the formula

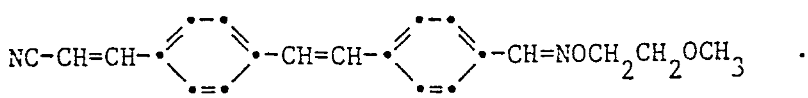

7. The stilbene compound according to claim 5 of the formula

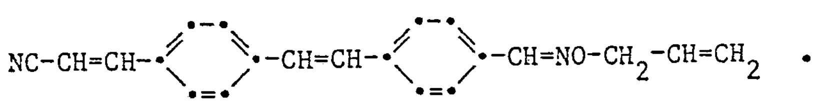

8. A process for the preparation of a stilbene compound of the formula

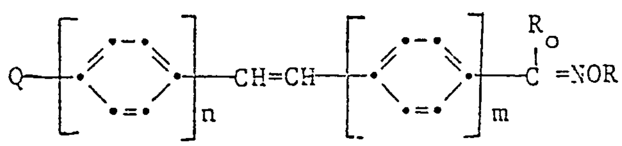 ,

wherein Q, R, $R_o$ m and n are as defined in claim 1, which process comprises reacting a carbonyl compound of the formula

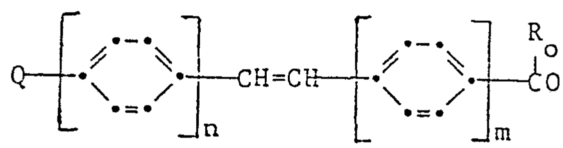 ,

with an O-substituted hydroxylamine of the formula

$H_2NOR$,

in an inert organic solvent.

9. A process for the preparation of a stilbene compound of the formula

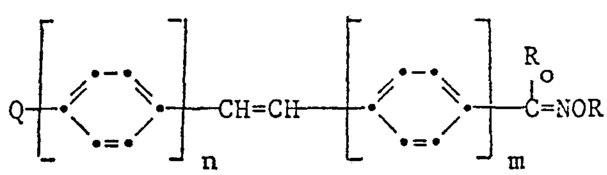 ,

wherein Q, R, $R_o$, m and n are as defined in claim 1, which process comprises reacting 1 mole of a compound of the formula

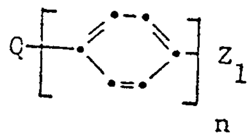

with 1 mole of a compound of the formula

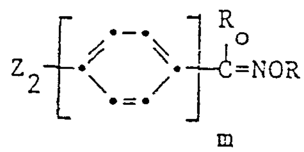

in the presence of a strong base and a polar solvent, in which formulae one of the symbols $Z_1$ and $Z_2$ is a HOC group and the other is a group of the formula

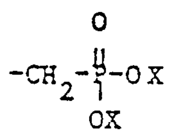 , 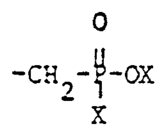 , 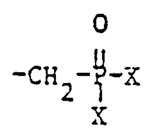 or 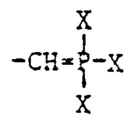

wherein X is an unsubstituted or substituted alkyl radical or 1 to 6 carbon atoms, an aryl radical, a cycloalkyl radical or an aralkyl radical.

10. A process for whitening manmade regenerated manmade or natural organic material of high molecular weight, which comprises incorporating in or applying to the surface of said material a stilbene compound as claimed in any one of claims 1 to 8.

11. A process according to claim 10 for whitening polyester fibres by the pad-heat or exhaust method.

12. A mixture containing a stilbene compound as claimed in any one of claims 1 to 8 and a fluorescent whitening agent which is known per se, in the ratio 1:9 to 9:1.

13. A mixture according to claim 12 containing a fluorescent whitening agent of the formula

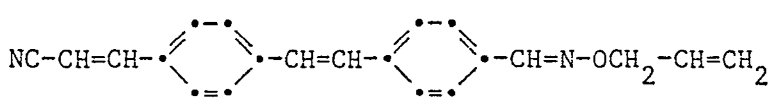

as stilbene compound.

14. A mixture according to claim I3 containing the fluorescent whitening agent of the formula

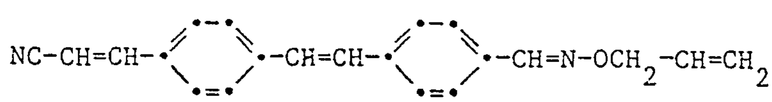

and the fluorescent whitening agent of the formula

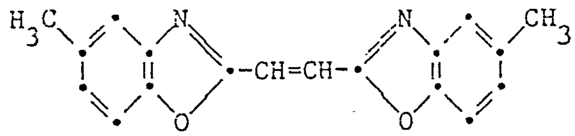

in the ratio 1:9 to 9:1.

**Revendications**

1. Composés stilbéniques de formule

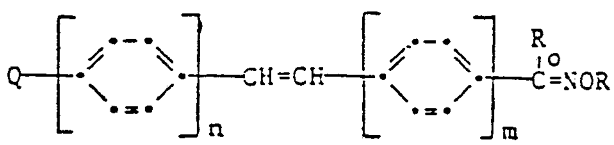

,

dans laquelle Q est l'hydrogène un système hétérocyclique aromatique monocyclique à 5 ou 6 chaînons non substitués ou substitués par des groupements non chromophores, et présentant O, 1 ou 2 noyaux benzéniques condensés, un système hétérocyclique aromatique bicyclique à 9 chaînons, ou bien le radical -CH = C($R_1$ )($R_2$ ), dans lequel $R_1$ represente l'hydrogène, ou un groupe alkyle en $C_{1-6}$ non substitué ou substituée par des groupements non chromophores, et $R_2$ represente un radical alcoxycarbonyle avec au total 2 à 7 atomes de carbone, cyano, carboxy, carbamoyle, dialkylphosphonate, (alkyle en $C_{1-6}$)-sulfonyle ou arylsulfonyle, ou bien Q peut encore signifier le groupe $-\underset{R_o}{\underset{|}{C}}=NOR$, $R_o$ étant l'hydrogene ou un groupe alkyle en $C_{1-6}$, et R étant un groupe alkyle en $C_{1-6}$, alcenyle en $C_{2-5}$, alcynyle en $C_{2-5}$, di-(alkyl en $C_{1-4}$)-aminoalkyle en $C_{1-4}$, hydroxyalkyle en $C_{1-4}$, phenyl-(alkyle en $C_{1-4}$), ou cycloalkyle avec un cycle de 5 à 12 chainons , non substitue ou substitue pardes groupementsnon chromophores et m et n représentent chacun indépendamment l'un de l'autre les nombres 1 ou 2.

2. Composés stilbéniques selon la revendication 1, de formule

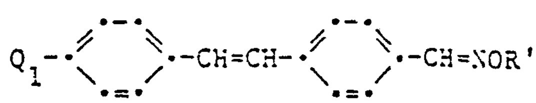

,

dans laquelle R' est un groupe alkyle en $C_{1-4}$, cyanoalkyle en $C_{1-4}$, (alcoxy en $C_{1-4}$)-alkyle en $C_{2-4}$, alcényle en $C_{3-4}$, alcynyle en $C_{3-4}$, ou un groupe de formule $R_xOOC$-(alkyle en $C_{1-4}$), ou $R_x$ représente l'hydrogène ou un groupe alkyle en $C_{1-4}$, et $Q_1$ est un groupe phényle ou le radical -CH = C($R_1$')($R_2$').

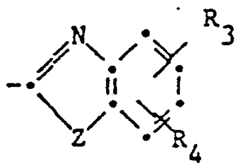, 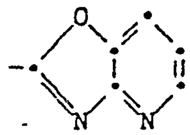, 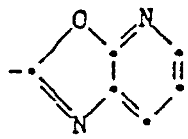,

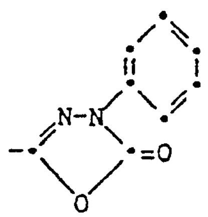, 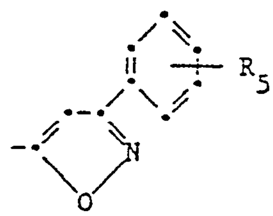, 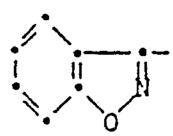, 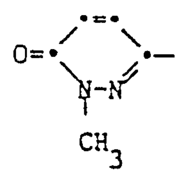,

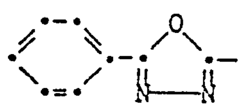, 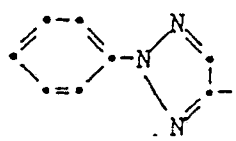, 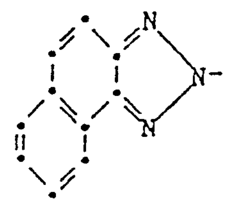

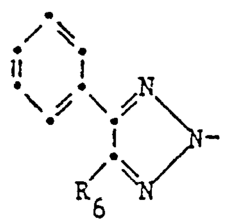 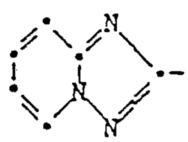 ou 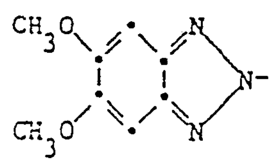

où $R_1$' est l'hydrogène ou un groupe alkyle en $C_{1-4}$, $R_2$' est un groupe alcoxycarbonyle avec au total de 2 à 5 atomes de carbone, cyano, carboxy, carbamoyle, (alkyle en $C_{1-4}$)-sulfonyle ou phénylsulfonyle, $R_3$ est l'hydrogène, le chlore, un groupe alkyle en $C_{1-4}$, un groupe phényl-(alkyle en $C_{1-3}$), cyclohexyle, phényle, alcoxy en $C_{1-4}$, (alkyle en $C_{1-4}$)-sulfonyle, (alcoxy en $C_{2-5}$)-carbonyle, cyano, ou carboxy, ou bien forme avec $R_4$4 un noyau condensé de benzène, de cyclopentane, ou de cyclohexane, non substitué de substitué par de 1 à 4 groupes méthyles, $R_4$ est l'hydrogène, le chlore, un groupe alkyle en $C_{-4}$ ou alcoxy en $C_{1-4}$ , ou bien forme avec $R_3$ un noyau condensé de benzène, de cyclopentane ou de cyclohexane, non substitué ou substitué par de 1 à 4 groupes méthyles, $R_5$ est l'hydrogene ou un groupe methoxy, $R_6$ est l'hydrogène, un groupe alkyle en $C_{1-4}$, ou un groupe alcoxycarbonyle avec au total de 2 à 5 atomes de carbone, et Z est O, S ou NX, X étant l'hydrogène, un groupe alkyle en $C_{1-4}$, ou un groupe phényle.

3. Composés stilbéniques selon la revendication 2, de formule

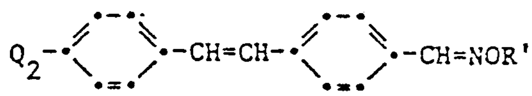

où R' a la signification indiquée dans la revendication 2, et $Q_2$ est le radical $-CH=C(R_1')(R_2'')$,

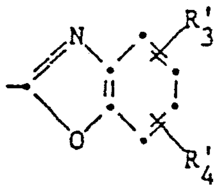

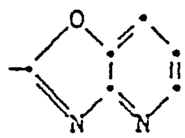 ou

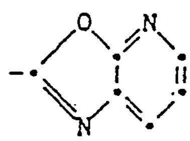

où

$R_1'$ est l'hydrogene ou un groupe alkyle en $C_{1-4}$, $R''_2$ est un groupe alcoxycarbonyle avec au total de 2 à 5 atomes de carbone ou un groupe cyano, $R'_3$ est l'hydrogène, un groupe alkyle en $C_{1-4}$, un groupe alcoxy en $C_{1-4}$, ou hien un groupe alcoxycarbonyle avec au total de 2 à 5 atomes de carbone, ou bien forme avec $R'_4$ un novau benzénique condense, et $R'_4$ est l'hydrogène ou un groupe alkyle en $C_{1-4}$, ou forme avec $R'_3$ un noyau benzénique condense.

4. Composés stilbéniques selon la revendication 3, de formule

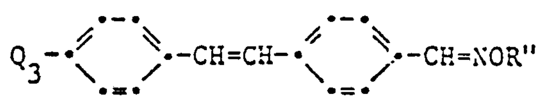 ,

où R" est un groupe alkyle en $C_{1-4}$ , un groupe alcoxy en $C_{1-4}$ )-alkyle en $C_{2-4}$ , ou alcényle en $C_{3-4}$, et$Q_3$ est le radical - CH=CH-CN, -CH=CH-COO-(alkyle en $C_{1-4}$) ou le radical

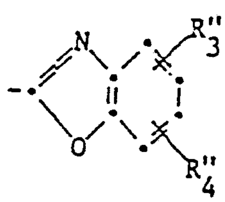

où $R''_3$ représente l'hydrogéne un groupe alkyle en $C_{1-4}$, alcoxy en $C_{1-4}$ ou alcoxycarbonyle en $C_{2-5}$ et $R''_4$ représente l'hydrogene ou un groupe alkyle en $C_{1-4}$.

5. Composés stilbéniques selon la revendication 4, de formule

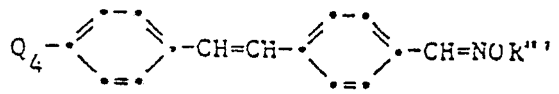,

où $R'''_3$ représente le grupe-$CH_3$,-$C_2H_5$-$C_2OCH_3$ ou -$CH_2$-$CH=CH_2$, et $Q_4$ le radical -CH=CH-CN, -CH=CH-COO-(alkyle en $C_{1-2}$)i ou

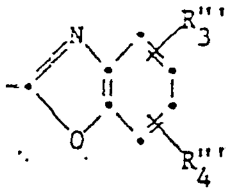

ou $R_3'''$ représente l'hydrogène ou un groupe alkyle en $C_{1-4}$, et $R_4'''$ représente l'hydrogène ou un groupe alkyle en $C_{1-4}$.

6. Le composé stilbénique selon la revendication 5, de formule

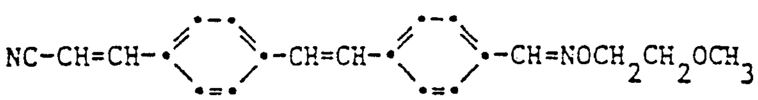.

7. Le composé stilbénique selon la revendication 5, de formule

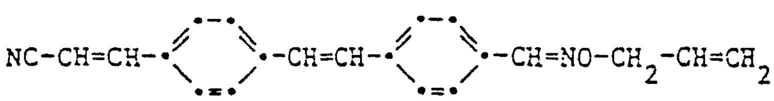

8. Procédé de préparation de composés stilbéniques de formule

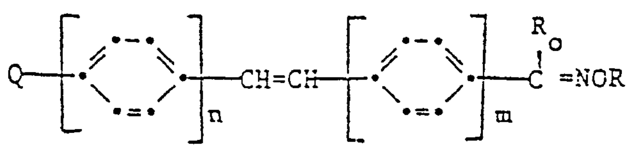,

où Q, R, $R_o$, m et n ont les significations données dans la revendication 1, caractérisé en ce que l'on fait réagir un composé carbonylé de formule

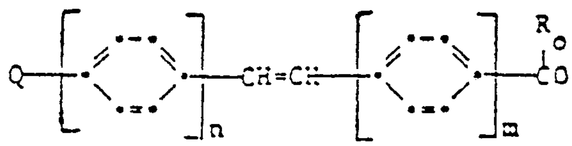

avec une hydroxylamine substituée sur l'oxygène de formule
$H_2NOR$
dans un solvant organique inerte.

9. Procéde de préparation de composés stilbéniques de formule

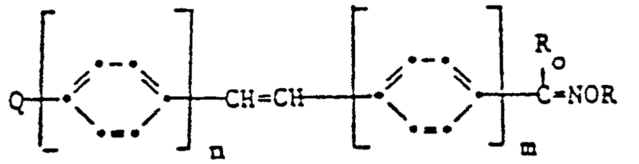 ,

où Q R $R_0$ m et n ont les significations données dans la revendication 1 caractérisé en ce que l'on fait reagir une mole d'un composé de formule

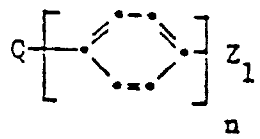

avec une mole d'un compose de formule

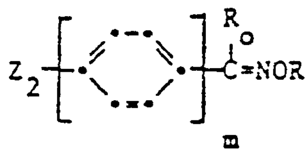

en présence d'une base forte et d'un solvant polaire, l'un des symboles $Z_1$ et $Z_2$ representant un groupe -CHO et l'autre un groupement de formule

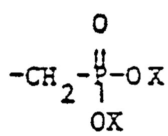 , 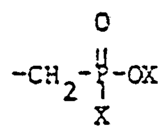 , 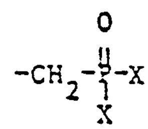 ou 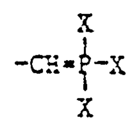

où X représente un radical alkyle, substitué ou non, possédant de 1 à 6 atomes de carbone, ou bien un radical aryle, cycloalkyle ou aralkyle.

10. Procéde d'azurage optique de matériaux organiques de poids moléculaire élevé, synthétiques, semi-synthétiques, ou naturels, caractérisé en ce que l'on incorpore à ces matériaux des composés stilbeniques tels que ceux définis dans l'une des revendications 1 à 6, ou bien on dépose de tels composés à la surface de ces matériaux.

11. Procédé selon la revendication 10, pour l'azurage optique de polvesters après le procédé d'éruisement et le procédé de foulardage et de thermofixation.

12. Mélange azurant contenant un composé stilbénique selon les revendications 1 à 6, et un azurant optique connu, dans un rapport de 1:9 à 9:1.

13. Mélange azurant selon la revendication 12, contenant l'azurant de formule

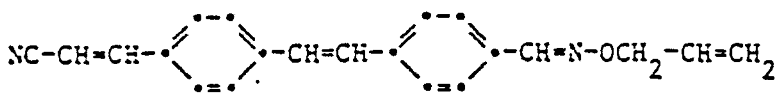

comme composé stilbénique.

14. Melange azurant selon la revendication 13, contenant l'azurant de formule

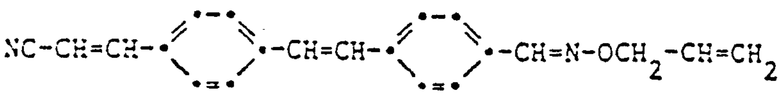

l'azurant de formule

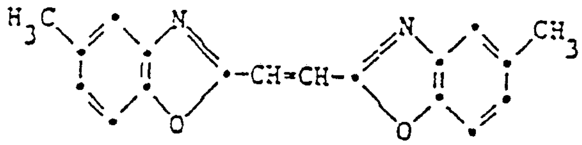

dans un rapport de 1:9 à 9:1.